# EUROPEAN PATENT SPECIFICATION

(11) **EP 3 768 159 B1**
(45) Date of publication and mention of the grant of the patent: **01.01.2025**
(21) Application number: 19771598.0
(22) Date of filing: 20.03.2019
(51) Int. Cl.: A61B 5/24, A61B 5/145, A61B 5/00, A61B 5/08

(54) **REPLACEABLE SENSOR SYSTEMS**
AUSTAUSCHBARE SENSORSYSTEME
SYSTÈMES DE CAPTEURS REMPLAÇABLES

(30) Priority: 20.03.2018 US 201862645565 P
(43) Date of publication of application: 27.01.2021
(60) Divisional application: 24205870.9
(73) Proprietor: Graphwear Technologies Inc., San Francisco, CA 94107 (US)
(72) Inventor: GUDIBANDE, Rajatesh Ravindra, San Francisco, California 94122 (US); RADHAKRISHNAN, Saurabh, San Francisco, California 94158 (US); VORA, Meet, San Francisco, California 94107 (US); GALAND, Antoine, San Francisco, California 94103 (US)
(74) Representative: Mewburn Ellis LLP
(86) International application number: PCT/US2019/023255
(87) International publication number: WO 2019/183279

(56) References cited:
- GB-A- 2 539 224
- US-A1- 2014 235 967
- US-A1- 2014 288 381
- US-A1- 2016 367 151
- US-A1- 2017 251 958
- US-A1- 2018 026 393
- US-B2- 9 592 007

## Description

### CROSS-REFERENCE

This application claims the benefit of U.S. Provisional Application No. 62/645,565, filed March 20, 2018.

### BACKGROUND

Non-invasive, quick, and convenient ways of sensing signals (e.g., physiological signals) is increasingly becoming an area of interest for healthcare, in the clinical setting or for general consumer markets. However, conventional monitoring systems may lack the ability (e.g., sensitivity, specificity, etc) to detect diseases and/or body physiology, or the convenience demanded in today's world. For example, invasive methods may be necessary, or blood or urine may be required for monitoring. Accordingly, there may be a need for non-invasive and effective ways to monitor body physiology or detect diseases, e.g., via sweat. Exemplary non-invasive sensor systems are known from US 2017/251958 A1, US 2014/288381 A1, GB 2 539 224 A and US 2018/026393 A1.

### SUMMARY

The invention is defined by the appended claims. Embodiments disclosed herein provide devices, systems, and methods (not covered by the invention) for monitoring physiological signals. Disposable or replaceable sensors may be utilized in monitoring physiological signals with high sensitivity and/or specificity. Various physiological signals, including glucose or lactic acid may be monitored conveniently, and in real time with no inconvenience to a user. For example, a user may wear device (e.g., a patch or a small attachment such as a wrist strap) anywhere on their body (e.g., as a wrist band) and the device may monitor and detect sweat to screen for physiological signals. Small disposable or replaceable sensors may beneficially be provided that may be coupled and uncoupled from the device such that signals may be monitored accurately and conveniently.

According to some aspects of the disclosure, a modular sensor is disclosed. The modular sensor may comprise: a substrate; a plurality of contact electrodes provided on a surface of the substrate; and a plurality of sensing lines disposed between the plurality of contact electrodes to collectively form a plurality of sensor elements, wherein each sensor element comprises at least one sensing line extending longitudinally between a pair of contact electrodes, wherein the modular sensor is configured to be operably and releasably coupled to a device for use as a sensing apparatus.

In some embodiments, the modular sensor is configured to function as an active sensing unit when electronically coupled to the device. In some embodiments, the modular sensor is configured to fit within a recessed housing on the device. In some embodiments, the modular sensor is protected by the recessed housing. In some embodiments, the substrate comprises a ferrous metal or alloy, and the device comprises a magnetic material. In some embodiments, the modular sensor is configured to be coupled and held in place on the device via an attractive force between the magnetic material and the ferrous metal or alloy.

At least one of the plurality of sensing lines comprises a nanoscale material. In some embodiments, at least one of the plurality of sensing lines comprises graphene. In some embodiments, the plurality of sensing lines each comprises graphene. In some embodiments, the plurality of sensor elements is configured to detect one or more markers in a fluid. In some embodiments, the plurality of sensor elements is configured to detect one or more biomarkers in a biological fluid of a subject. In some embodiments, the plurality of sensor elements is configured to detect a same biomarker.

In some embodiments, the biological fluid comprises sweat or interstitial fluid obtained via the surface of the skin. In some embodiments, the biological fluid comprises breath or lung originated water vapor obtained from exhaling on the device. In some embodiments, each of the plurality of sensor elements is configured to detect a different biomarker. In some embodiments, the plurality of sensor elements is configured operate in a multichannel multiplexed configuration. In some embodiments, the one or more biomarkers comprises an electrolyte, glucose, lactic acid, IL6, a cytokine, HER2, Cortisol, ZAG, cholesterol, vitamins, a protein, a drug molecule, a metabolite, a peptide, an amino acid, a DNA, an RNA, an aptamer, an enzyme, a biomolecule, a chemical molecule, a synthetic molecule, or combinations thereof. In some embodiments, the one or more biomarkers comprises an electrolyte, glucose, and lactic acid.

In some embodiments, the biological fluid sample comprises sweat, breath, saliva, earwax, urine, semen, blood plasma, a bio-fluid, a chemical fluid, an air sample, a gas sample, or a combination thereof. In some embodiments, the biological fluid sample comprises sweat or breath. In some embodiments, the plurality of sensor elements is configured to detect the one or more biomarkers when in contact with the biological fluid sample. In some embodiments, the plurality of sensor elements is capable of detecting the one or more biomarkers in a non-invasive manner, without requiring penetration of the subject's skin to extract the biological fluid sample.

In some embodiments, the plurality of sensor elements is configured to detect a presence and concentration of the one or more biomarkers substantially in real-time when the device is being worn on the subject or in proximity to the subject. In some embodiments, data indicative of the presence and concentrations of the one or more biomarkers is collected and stored by the device. In some embodiments, the data is collected and stored on the device over a time period that the device is being worn on the subject or in proximity to the subject. In some embodiments, the modular sensor is configured to be operably and releasably coupled to the device without the use of tools. In some embodiments, the modular sensor is configured to be operably and releasably coupled to the device in less than 10 seconds.

Also disclosed is a sensing apparatus (not part of the invention).

A sensing apparatus may comprise: a plurality of modular sensors configured to detect one or more biomarkers in a biological fluid sample of a subject when the device is being worn by the subject or in proximity to the subject; and a device configured to interchangeably and releasably couple to a modular sensor selected from the plurality of modular sensors, wherein the device is configured to receive,store, and send sensing signals from the modular sensor.

In some embodiments, the device comprises a transmitter configured to transmit the sensing signals over a network. In some embodiments, the transmitter is configured to transmit the sensing signals to a mobile device that is associated with and in proximity to the subject. In some embodiments, the device comprises a recessed housing configured to receive and support the modular sensor. In some embodiments, the device is releasably coupled to the modular sensor via a magnetic attachment mechanism. In some embodiments, the magnetic attachment mechanism comprises a magnetic material provided on at least one of the modular sensor and the device, and a ferrous metal or alloy provided on at least one of the modular sensor and the device. In some embodiments, the device is configured to be releasably coupled to a strap or patch, wherein the strap or patch is configured to be worn on a portion of the subject's body. In some embodiments, the plurality of modular sensors comprises at least one graphene-based sensor.

Also disclosed is a device (not part of the invention).

The device may comprise: a processing module configured to operably couple to at least one sensor selected from a group consisting of a plurality of discrete biological or chemical sensors, wherein two or more different sensors for detecting two or more different target analytes are interchangeably and releasably attachable to the device, depending on a type(s) of target analytes to be detected from a sample of a subject collected on the device when the subject is wearing the device or in proximity to the device.

In some embodiments, the sample comprises sweat, saliva, breath, blood, or other biological fluids of the subject. In some embodiments, the different target analytes comprise different biomarkers and/or chemical agents. In some embodiments, the biomarkers are selected from the group consisting of electrolytes, glucose, and lactic acid. In some embodiments, at least one of the sensors is configured to measure a pH or ionic concentration of the sample. In some embodiments, at least one of the sensors comprises a graphene-based sensor. In some embodiments, the plurality of discrete sensors are heterogeneous sensors comprising (i) at least one graphene-based sensor and (ii) at least one non graphene-based sensor. In some embodiments, the processing module is configured to detect and monitor levels of a first target analyte when a first sensor specific to the first target analyte is attached to the device. In some embodiments, the processing module is configured to switch to detection and monitoring of a second target analyte when the first sensor is detached from the device and replaced by a second sensor specific to the second target analyte. In some embodiments, the processing module is located onboard the device, and configured to process sensor data substantially in real-time as the data is being collected by the at least one sensor, in order to detect and monitor levels of one or more target analytes. In some embodiments, the device comprises a graphical display for displaying the detected levels of the one or more target analytes. In some embodiments, the processing module is configured to transmit the processed sensor data to a remote device, server or third party entity. In some embodiments, the processing module comprises a recommendation engine configured to prescribe certain corrective or mitigative actions to the subject, based on the detected levels of the one or more target analytes.

In some embodiments (not part of the invention), a modular sensing kit is disclosed. The modular sensing kit may comprise (1) the device and (2) the plurality of discrete biological or chemical sensors of claim on any aspect or embodiment. In some embodiments, a quick release mechanism provided on the device allows different discrete sensors to be manually attached and detached from the device without the use of tools. In some embodiments, the plurality of discrete sensors are provided separately from the device. In some embodiments, one or more of the discrete sensors is configured for a single use with the device, and discarded after each use encounter by the subject. In some embodiments, one or more of the discrete sensors is configured for multiple uses with the device, and capable of being recycled and reused in multiple use encounters by the subject. In some embodiments, the plurality of discrete sensors have different sensitivities to a same target analyte or different target analytes. In some embodiments, the plurality of discrete sensors comprises a first sensor and a second sensor both configured to detect a target analyte, wherein the first sensor has a higher sensitivity than the second sensor. In some embodiments, the first sensor is capable of detecting a substantially lower level or concentration of the target analyte compared to the second sensor.

Also disclosed is a device (not part of the invention). The device may comprise: a processing module operably coupled to three or more different discrete biological or chemical sensors, wherein the processing module is configured to selectively activate the three or more different discrete biological or chemical sensors in different multiplexed configurations depending on desired type(s) of sensing application of a subject.

In some embodiments, the different multiplexed configurations permit a plurality of different target analytes to be detected from a sample of the subject collected on the device when the subject is wearing the device or in proximity to the device. In some embodiments, the different multiplexed configurations enable increased sensitivity in the detection and monitoring of different target analytes. In some embodiments, the processing module is configured to selectively activate a fewer number of the biological or chemical sensors to reduce power consumption of the device. In some embodiments, the processing module is configured to selectively activate a greater number of the biological or chemical sensors to enhance sensitivity in the detection and monitoring of different target analytes. In some embodiments, the three or more discrete sensors comprises a first sensor for detecting a first target analyte, a second sensor for detecting a second target analyte, and a third sensor for detecting a third target analyte. In some embodiments, the processing module is configured to selectively activate at least two out of the first, second and third sensors. In some embodiments, the processing module is configured to selectively activate (1) the first and second sensors in a first multiplexed configuration, (2) the second and third sensors in a second multiplexed configuration, or (3) the first and third sensors in a third multiplexed configuration. In some embodiments, the processing module is capable of detecting (1) the presence and (2) concentrations ranging from 1 fg/L and above of two or more different target analytes in a sample having a volume of less than 1 µL collected from the subject on the device when the subject is wearing the device or in proximity to the device. In some embodiments, the device is capable of detecting the presence and concentrations of the two or more different target analytes in less than 1 second.

Also disclosed is a method of fabricating a modular sensor (the method being not part of the invention). The method may comprise:
providing a sensor substrate comprising at least two electrodes disposed on a surface of the substrate; depositing a layer of graphene on the surface of the sensor substrate between the at least two electrodes; metallizing at least a portion of the layer of graphene at or near the at least two electrodes; passivating at least a portion of the layer of graphene with a passivation polymer; and optionally, functionalizing at least a portion of the layer of graphene, wherein functionalizing the layer of graphene with a receptor layer, wherein the receptor layer is sensitive to a target analyte.

In some embodiments, the receptor layer comprises a receptor selected from group consisting of pyrene boronic acid (PBA), pyrene N-hydroxysuccinimide ester (Pyrene-NHS), organic chemicals, aromatic molecules, cyclic molecules, enzymes, proteins, antibodies, viruses, single stranded DNAs (ssDNAs), aptamers, inorganic materials, synthetic molecules, and biological molecules. In some embodiments, the target analyte comprises an electrolyte, glucose, lactic acid, IL6, a cytokine, HER2, Cortisol, ZAG, cholesterol, vitamins, a protein, a drug molecule, a metabolite, a peptides, an amino acid, a DNA, an RNA, an aptamer, an enzyme, a biomolecule, a chemical molecule, a synthetic molecule, or combinations thereof. In some embodiments, the substrate comprises polyamide, Polyethylene terephthalate (PET), polydimethylsiloxane (PDMS), Poly(methyl methacrylate) (PMMA), other plastics, silicon dioxide, silicon, glass, aluminum oxide, sapphire, germanium, gallium arsenide, indium phosphide, an alloy of silicon and germanium, fabrics, textiles, silk, paper, cellulose based materials, insulator, metal, semiconductor, or a combination thereof. In some embodiments, the substrate is flexible. In some embodiments, the passivation polymer comprises Acrylic, PMMA, silicone, polysilicone, PDMS, rubber, hotmelt co-polymers, EVA co polymers, ethylene acrylate, PET, Polyamide, PTFE, fluoropolymer, thermoplastics, gels, hydrogels, polypropylene, polyethylene, Polyolefins, polyvinyl chloride, polyesters, polyurethanes, Styrene block copolymers, Polycaprolactone, Polycarbonates, Fluoropolymers, Silicone rubbers, Thermoplastic elastomers, Polypyrrole, or a combination thereof. In some embodiments, the passivation polymer is polyurethane. In some embodiments, the depositing the graphene layer comprises heating the substrate beyond a fusing temperature of a functional back polymer disposed between the graphene layer and the substrate. In some embodiments, the method further comprises functionalizing a first portion of the substrate near the graphene layer with a hydrophilic material. In some embodiments, a second portion of the substrate near the graphene layer is not functionalized with the hydrophilic material. In some embodiments, the second portion of the substrate is functionalized with a hydrophobic material.

According to some aspects of the disclosure, a modular sensor is provided. The modular sensor may comprise a substrate; a plurality of contact electrodes provided on a surface of the substrate; and a plurality of sensing lines disposed between the plurality of contact electrodes to collectively form a plurality of sensor elements, wherein each sensor element comprises at least one sensing line extending longitudinally between a pair of contact electrodes, wherein the modular sensor is configured to be operably and releasably coupled to a device for use as a wearable sensing apparatus.

In some embodiments, the modular sensor is configured to function as an active sensing unit when electronically coupled to the device. In some embodiments, the modular sensor is configured to fit within a recessed housing on the device. In some embodiments, the modular sensor is protected by the recessed housing when the device is being worn by a subject. In some embodiments, the substrate comprises a ferrous metal or alloy, and the device comprises a magnetic material. In some embodiments, the modular sensor is configured to be coupled and held in place on the device via an attractive force between the magnetic material and the ferrous metal or alloy. In some embodiments, at least one of the plurality of sensing lines comprises graphene. In some embodiments, the plurality of sensing lines each comprises graphene.

In some embodiments, the plurality of sensor elements is configured to detect one or more biomarkers in a biological fluid sample of a subject when the device is being worn by the subject. In some embodiments, the plurality of sensor elements is configured to detect a same biomarker. In some embodiments, each of the plurality of sensor elements is configured to detect a different biomarker. In some embodiments, the plurality of sensor elements is configured operate in a multichannel multiplexed configuration. In some embodiments, the one or more biomarkers comprises an electrolyte, glucose, lactic acid, IL6, a cytokine, HER2, Cortisol, ZAG, cholesterol, vitamins, a protein, a drug molecule, a metabolite, a peptide, an amino acid, a DNA, an RNA, an aptamer, an enzyme, a biomolecule, a chemical molecule, a synthetic molecule, or combinations thereof. In some embodiments, the one or more biomarkers comprises an electrolyte, glucose, and lactic acid. In some embodiments, the biological fluid sample comprises sweat, breath, saliva, earwax, urine, semen, blood plasma, a bio-fluid, a chemical fluid, an air sample, a gas sample, or a combination thereof. In some embodiments, the biological fluid sample comprises sweat or breath. In some embodiments, the plurality of sensor elements is configured to detect the one or more biomarkers when in contact with the biological fluid sample.

In some embodiments, the plurality of sensor elements is capable of detecting the one or more biomarkers in a non-invasive manner, without requiring penetration of the subject's skin to extract the biological fluid sample. In some embodiments, the plurality of sensor elements is configured to detect a presence and concentration of the one or more biomarkers substantially in real-time when the device is being worn on the subject. In some embodiments, data indicative of the presence and concentrations of the one or more biomarkers is collected and stored by the device. In some embodiments, the data is collected and stored on the device over a time period that the device is being worn on the subject. In some embodiments, the modular sensor is configured to be operably and releasably coupled to the device without the use of tools. In some embodiments, the modular sensor is configured to be operably and releasably coupled to the device in less than 10 seconds.

Also disclosed is a wearable sensing apparatus (not part of the invention).

The wearable sensing apparatus may comprise a plurality of modular sensors configured to detect one or more biomarkers in a biological fluid sample of a subject when the device is being worn by the subject; and a device configured to interchangeably and releasably couple to a modular sensor selected from the plurality of modular sensors, wherein the device is configured to receive and store sensing signals from the modular sensor.

In some embodiments, the device comprises a transmitter configured to transmit the sensing signals over a network. In some embodiments, the transmitter is configured to transmit the sensing signals to a mobile device that is associated with and in proximity to the subject. In some embodiments, the device comprises a recessed housing configured to receive and support the modular sensor. In some embodiments, the device is releasably coupled to the modular sensor via a magnetic attachment mechanism. In some embodiments, the magnetic attachment mechanism comprises a magnetic material provided on at least one of the modular sensor and the device, and a ferrous metal or alloy provided on at least one of the modular sensor and the device. In some embodiments, the device is configured to be releasably coupled to a strap, wherein the strap is configured to be worn on a portion of the subject's body. In some embodiments, the plurality of modular sensors comprises at least one graphene-based sensor.

Also disclosed is a wearable device (not part of the invention).

The wearable device may comprise a processing module configured to operably couple to at least one sensor selected from a group consisting of a plurality of discrete biological or chemical sensors, wherein two or more different sensors for detecting two or more different target analytes are interchangeably and releasably attachable to the wearable device, depending on a type(s) of target analytes to be detected from a sample of a subject collected on the wearable device when the subject is wearing the device.

In some embodiments, the sample comprises sweat, saliva, breath, blood, or other biological fluids of the subject. In some embodiments, the different target analytes comprise different biomarkers and/or chemical agents. In some embodiments, the biomarkers are selected from the group consisting of electrolytes, glucose, and lactic acid. In some embodiments, at least one of the sensors is configured to measure a pH or ionic concentration of the sample. In some embodiments, at least one of the sensors comprises a graphene-based sensor. In some embodiments, the plurality of discrete sensors are heterogeneous sensors comprising (i) at least one graphene-based sensor and (ii) at least one non graphene-based sensor.

In some embodiments, the processing module is configured to detect and monitor levels of a first target analyte when a first sensor specific to the first target analyte is attached to the wearable device. In some embodiments, the processing module is configured to switch to detection and monitoring of a second target analyte when the first sensor is detached from the wearable device and replaced by a second sensor specific to the second target analyte. In some embodiments, the processing module is located onboard the wearable device, and configured to process sensor data substantially in real-time as the data is being collected by the at least one sensor, in order to detect and monitor levels of one or more target analytes. In some embodiments, the wearable device comprises a graphical display for displaying the detected levels of the one or more target analytes. In some embodiments, the processing module is configured to transmit the processed sensor data to a remote device, server or third party entity. In some embodiments, the processing module comprises a recommendation engine configured to prescribe certain corrective or mitigative actions to the subject, based on the detected levels of the one or more target analytes.

Also disclosed is a modular sensing kit (not part of the invention).

The modular sensing kit may comprise (1) the wearable device and (2) the plurality of discrete biological or chemical sensors of any embodiment disclosed herein. In some embodiments, a quick release mechanism provided on the wearable device allows different discrete sensors to be manually attached and detached from the wearable device without the use of tools. In some embodiments, the plurality of discrete sensors are provided separately from the wearable device. In some embodiments, one or more of the discrete sensors is configured for a single use with the wearable device, and discarded after each use encounter by the subject. In some embodiments, one or more of the discrete sensors is configured for multiple uses with the wearable device, and capable of being recycled and reused in multiple use encounters by the subject. In some embodiments, the plurality of discrete sensors have different sensitivities to a same target analyte or different target analytes. In some embodiments, the plurality of discrete sensors comprises a first sensor and a second sensor both configured to detect a target analyte, wherein the first sensor has a higher sensitivity than the second sensor. In some embodiments, the first sensor is capable of detecting a substantially lower level or concentration of the target analyte compared to the second sensor.

Also disclosed is a wearable device (not part of the invention).

The wearable device may comprise a processing module operably coupled to three or more different discrete biological or chemical sensors, wherein the processing module is configured to selectively activate the three or more different discrete biological or chemical sensors in different multiplexed configurations depending on desired type(s) of sensing application of a subject. In some embodiments, the different multiplexed configurations permit a plurality of different target analytes to be detected from a sample of the subject collected on the wearable device when the subject is wearing the device. In some embodiments, the different multiplexed configurations enable increased sensitivity in the detection and monitoring of different target analytes. In some embodiments, the processing module is configured to selectively activate a fewer number of the biological or chemical sensors to reduce power consumption of the wearable device. In some embodiments, the processing module is configured to selectively activate a greater number of the biological or chemical sensors to enhance sensitivity in the detection and monitoring of different target analytes. In some embodiments, the three or more discrete sensors comprises a first sensor for detecting a first target analyte, a second sensor for detecting a second target analyte, and a third sensor for detecting a third target analyte.

In some embodiments, the processing module is configured to selectively activate at least two out of the first, second and third sensors. In some embodiments, the processing module is configured to selectively activate (1) the first and second sensors in a first multiplexed configuration, (2) the second and third sensors in a second multiplexed configuration, or (3) the first and third sensors in a third multiplexed configuration. In some embodiments, the processing module is capable of detecting (1) the presence and (2) concentrations ranging from 1 femtogram per liter (fg/L) and above of two or more different target analytes in a sample having a volume of less than 1 microliters (µL) collected from the subject on the wearable device when the subject is wearing the device. In some embodiments, the wearable device is capable of detecting the presence and concentrations of the two or more different target analytes in less than 1 second.

Also disclosed is a method of fabricating a modular sensor (the method being not part of the invention). The method may comprise:
providing a sensor substrate comprising at least two electrodes disposed on a surface of the substrate; depositing a layer of graphene on the surface of the sensor substrate between the at least two electrodes; metallizing at least a portion of the layer of graphene at or near the at least two electrodes; passivating at least a portion of the layer of graphene with a passivation polymer; and optionally, functionalizing at least a portion of the layer of graphene, wherein functionalizing the layer of graphene with a receptor layer, wherein the receptor layer is sensitive to a target analyte.

In some embodiments, the receptor layer comprises a receptor selected from group consisting of pyrene boronic acid (PBA), pyrene N-hydroxysuccinimide ester (Pyrene-NHS), organic chemicals, aromatic molecules, cyclic molecules, enzymes, proteins, antibodies, viruses, single stranded DNAs (ssDNAs), aptamers, inorganic materials, synthetic molecules, and biological molecules. In some embodiments, the target analyte comprises an electrolyte, glucose, lactic acid, IL6, a cytokine, HER2, Cortisol, ZAG, cholesterol, vitamins, a protein, a drug molecule, a metabolite, a peptides, an amino acid, a DNA, an RNA, an aptamer, an enzyme, a biomolecule, a chemical molecule, a synthetic molecule, or combinations thereof. In some embodiments, the substrate comprises polyamide, Polyethylene terephthalate (PET), polydimethylsiloxane (PDMS), Poly(methyl methacrylate) (PMMA), other plastics, silicon dioxide, silicon, glass, aluminum oxide, sapphire, germanium, gallium arsenide, indium phosphide, an alloy of silicon and germanium, fabrics, textiles, silk, paper, cellulose based materials, insulator, metal, semiconductor, or a combination thereof. In some embodiments, the substrate is flexible. In some embodiments, the passivation polymer comprises Acrylic, PMMA, silicone, polysilicone, PDMS, rubber, hotmelt co-polymers, EVA co polymers, ethylene acrylate, PET, Polyamide, PTFE, fluoropolymer, thermoplastics, gels, hydrogels, polypropylene, polyethylene, Polyolefins, polyvinyl chloride, polyesters, polyurethanes, Styrene block copolymers, Polycaprolactone, Polycarbonates, Fluoropolymers, Silicone rubbers, Thermoplastic elastomers, Polypyrrole, or a combination thereof. In some embodiments, the passivation polymer is polyurethane.

In some embodiments, the depositing the graphene layer comprises heating the substrate beyond a fusing temperature of a functional back polymer disposed between the graphene layer and the substrate. In some embodiments, the method further comprises functionalizing a first portion of the substrate near the graphene layer with a hydrophilic material. In some embodiments, a second portion of the substrate near the graphene layer is not functionalized with the hydrophilic material. In some embodiments, the second portion of the substrate is functionalized with a hydrophobic material.

Accordingly, in one aspect, a disposable sensor may be provided. The disposable sensor may comprise: a substrate; two or more contact electrodes disposed on a surface of the substrate; and a sensor element disposed between the two or more contact electrodes, wherein the substrate comprises a volume equal to or less than about 5 cm³.

In some embodiments, the volume is equal to or less than about 0.5 cm³. In some embodiments, the sensor element comprises graphene. In some embodiments, the sensor is configured to detect glucose, lactic acid, or other biomarkers. In some embodiments, the sensor is configured to contact sweat, saliva, or breath to screen for disease or micronutrient information. In some embodiments, the sensor comprises a contact area configured to come into contact with a user's fingers. In some embodiments, the sensor comprises magnets.

In another aspect, a transmitter may be provided. The transmitter may comprise: a receiving port for receiving a disposable sensor, wherein the receiving port comprises a mechanism for coupling to the disposable sensor; a processor operably coupled to the receiving port; and an outer housing.

In some embodiments, the mechanism comprises magnets. In some embodiments, the transmitter comprises a volume equal to or less than about 100 cm3. In some embodiments, the transmitter comprises a volume equal to or less than about 50 cm3. In some embodiments, the transmitter comprises a coupling mechanism for coupling with a strap. In some embodiments, the strap is a wrist strap. In some embodiments, the processor is configured to receive signals from the disposable sensor and screen for disease or micronutrient information. In some embodiments, the processor is configured to screen for disease or micronutrient information in real time.

In another aspect, a system for sensing signals is provided (not part of the invention). The system may comprise: a disposable sensor; a transmitter comprising a sensor receiving portion for receiving the disposable sensor; and an attachment comprising a transmitter receiving portion for receiving the transmitter.

The invention is defined by the appended claims.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG. 1A** illustrates a transmitter module comprising a sensor of the present disclosure may be integrated into a wearable attachment in the form of an armband, in accordance with some embodiments;
**FIG. 1B** illustrates a transmitter module comprising a sensor of the present disclosure which may be integrated into a wearable attachment in the form of a patch, in accordance with some embodiments;
**FIG. 1C** illustrates a transmitter module comprising a sensor of the present disclosure can be integrated with a patch placed on the back of connected device, in accordance with some embodiments;
**FIG. 2** shows an isometric view of a sensor, in accordance with some embodiments;
**FIG. 3A** illustrates a top view of a sensor, in accordance with some embodiments;
**FIG. 3B** illustrates a side view of a sensor, in accordance with some embodiments;
**FIG. 3C** illustrates a bottom view of a sensor, in accordance with some embodiments;
**FIG. 4A, FIG. 4B,** and **FIG. 4C** illustrate an additional example of a sensor, in accordance with some embodiments;
**FIG. 5A** and **FIG. 5B** illustrate an example of a sensor substrate with a single sensor element and an example of a sensor substrate with multiplexed sensor elements, in accordance with some embodiments;
**FIG. 6A** illustrates a first example of a transmitter module, in accordance with embodiments;
**FIG. 6B** illustrates a top view of a transmitter module, in accordance with some embodiments;
**FIG. 6C** illustrates a side view of a transmitter module, in accordance with some embodiments;
**FIG. 6D** illustrates a bottom view of a transmitter module without a sensor mounted thereon, in accordance with some embodiments;
**FIG. 6E** illustrates a bottom view of a transmitter with a sensor module mounted thereon, in accordance with some embodiments;
**FIG. 7A** illustrates an exploded view of the interior of a transmitter module, in accordance with some embodiments;
**FIG. 7B** illustrates a side view of an interior of a transmitter along a slice through the transmitter, in accordance with some embodiments;
**FIG. 7C** illustrates an isometric view of a transmitter with a top housing removed, in accordance with some embodiments;
**FIG. 7D** illustrates a top view of a transmitter with a top housing removed, in accordance with some embodiments;
**FIG. 7E** illustrates a side view of a transmitter with top housing removed, in accordance with some embodiments;
**FIG. 8A, FIG. 8B,** and **FIG. 8C** illustrate a second example transmitter module, in accordance with some embodiments,
**FIG. 9A** illustrates an exploded view of the interior of a second example of a transmitter module, in accordance with some embodiments;
**FIG. 9B** illustrates a side view of an interior of a second example of a transmitter module along a slice through the transmitter, in accordance with some embodiments;
**FIG. 10A** illustrates a transmitter module comprising a sensor of the present disclosure integrated into a wearable arm band, in accordance with some embodiments;
**FIG. 10B** illustrates a bottom view of a biosensing system, in accordance with some embodiments;
**FIG. 10C** illustrates a transmitter module of the present disclosure being removable from the wearable arm band, in accordance with some embodiments;
**FIG. 10D** illustrates a wearable arm band with strap mechanism engaged, in accordance with some embodiments;
**FIG. 11A, FIG. 11B,** and **FIG. 11C** illustrate a second example of a wearable arm band, in accordance with some embodiments;
**FIG. 12A** illustrates a top view of an example of a patch mount with a transmitter module mounted thereon, in accordance with some embodiments;
**FIG. 12B** illustrates an example of a patch mount with a transmitter module decoupled from the patch mount, in accordance with some embodiments;
**FIG. 12C** illustrates a view of an integrated sensor patch system, in accordance with some embodiments;
**FIG. 13A, FIG. 13B, FIG. 13C,** and **FIG. 13D** illustrate an example of a patch system which may be coupled to a transmitter module which is also coupleable to an arm band, in accordance with some embodiments;
**FIG. 14** shows a wearable device of the present disclosure in connection with two connected devices, in accordance with some embodiments;
**FIG. 15** illustrates an example connected device programmed or otherwise configured to interface with a transmitter module, in accordance with some embodiments;
**FIG. 16A** shows a transmitter module decoupled from a docking station, in accordance with some embodiments;
**FIG. 16B** shows a transmitter module coupled from a docking station, in accordance with some embodiments;
**FIG. 17A** shows a transmitter module decoupled from a docking station, in accordance with some embodiments; and
**FIG. 17B** shows a transmitter module coupled from a docking station, in accordance with some embodiments.

### DETAILED DESCRIPTION

The invention is defined by the appended claims.

Non-invasive, quick, and convenient ways of sensing signals (e.g., physiological signals) may be desired. For example, there is a need for screening for diseases and/or generally monitoring body physiology without blood or urine for the general population, patients (e.g., diabetics), and athletes. The present disclosure provides devices, systems, and methods for monitoring for physiological signals non-invasively, quickly, and conveniently with high sensitivity and/or specificity. In one example, the systems or devices provided herein may sense biomarkers such as glucose level or body osmolality. For instance, the systems or devices may non-invasively measure glucose and/or electrolytes in real time from sweat, or other bodily fluids such as saliva.

Systems and methods of the present disclosure may detect a biological fluid. In some examples, the biological fluid comprises a solution with polar molecules, a gas with polar molecules, a target sensing analyte, or combinations thereof. In some examples, the biological fluid comprises sweat, breath, saliva, earwax, urine, semen, blood plasma, interstitial fluid, lung-originated water vapor, a bio-fluid, a chemical fluid, an air sample, a gas sample, or a combination thereof. In some embodiments, the target analyte comprises an electrolyte, glucose, lactic acid, IL6, a cytokine, HER2, Cortisol, ZAG, cholesterol, vitamins, a protein, a drug molecule, a metabolite, a peptide, an amino acid, a DNA, an RNA, an aptamer, an enzyme, a biomolecule, a chemical molecule, a synthetic molecule, or combinations thereof.

The systems or devices may further be worn unobtrusively (e.g., everyday) in convenient patch or strap form factors. The devices may be worn, synced (e.g., with a server), and be used to track in real time the health or general physical state of the user. The user may utilize the information provided by the devices to take further action as desired.

The system may use Bluetooth to transmit data and may interact with a user, for example, with a screen or one or more light emitting diodes (LEDs). The transmitter module of the system may comprise a processing module and optionally an outer housing. The housing may house a sensor write/readout assembly, associated electronics, communications devices and/or magnets to facilitate attachment of a sensor to the transmitter.

The sensor may be removably coupled to the transmitter. The sensor a sensor substrate, electrodes, and sensor elements such as graphene. The weight of the biosensing system may be negligible, for example, equal to or less than about 500g, 400g, 300g, 200g, 150g, 120g, 100g, 80g, 60g, 40g, 30g, 20g, 10g, 5g, 4g, 3g, 2g, or 1g. Optionally, the sensor may be a disposable sensor. Alternatively or in addition, the sensor may be a replaceable sensor. For example, the sensor may be used, cleaned or processed, and be used again. While disposable sensors are primarily discussed herein, it is to be understood that details and/or descriptions discussed with respect to disposable sensors may be applicable to replaceable sensors.

As described throughout, the systems, devices, and methods provide non-invasive, quick, and convenient ways of sensing signals. This may be provided by a number of factors, including, but not limited to one or more of: 1) a replaceable magnetic sensor substrate, 2) a flexible printed circuit material with embedded sensor electrodes and metal vias folded around a magnetic iron sheet, 3) a sensor substrate with alternate hydrophobic and hydrophilic regions to facilitate sweat localization/absorption, 4) a metal contact and passivation layer thickness and method of application, 5) a dual conduction layer strategy - graphene, conduction layer 1, cure, conduction layer 2 and then passivation, 6) a constant current 5 to 200 micro Amps, across sensor and voltage readout 8 bit or higher/or 7) a wearable mount: band and patch.

### BIOSENSOR

A biosensor of the present disclosure may detect a biological fluid. In some examples, the biological fluid comprises a solution with polar molecules, a gas with polar molecules, a target sensing analyte, or combinations thereof. In some examples, the biological fluid comprises sweat, breath, saliva, earwax, urine, semen, blood plasma, interstitial fluid, lung-originated water vapor, a bio-fluid, a chemical fluid, an air sample, a gas sample, or a combination thereof. In some embodiments, the target analyte comprises an electrolyte, glucose, lactic acid, IL6, a cytokine, HER2, Cortisol, ZAG, cholesterol, vitamins, a protein, a drug molecule, a metabolite, a peptide, an amino acid, a DNA, an RNA, an aptamer, an enzyme, a biomolecule, a chemical molecule, a synthetic molecule, or combinations thereof.

In some cases, a biosensor of the present disclosure may be portable. In some cases, a biosensor may be in proximity to subject. In some cases, a biosensor may be carried by a user. In some cases, a biosensor may be attached to a connected device, as disclosed elsewhere herein, which may be carried by a user. In some cases, the biosensor is a wearable device which may be coupled to a user.

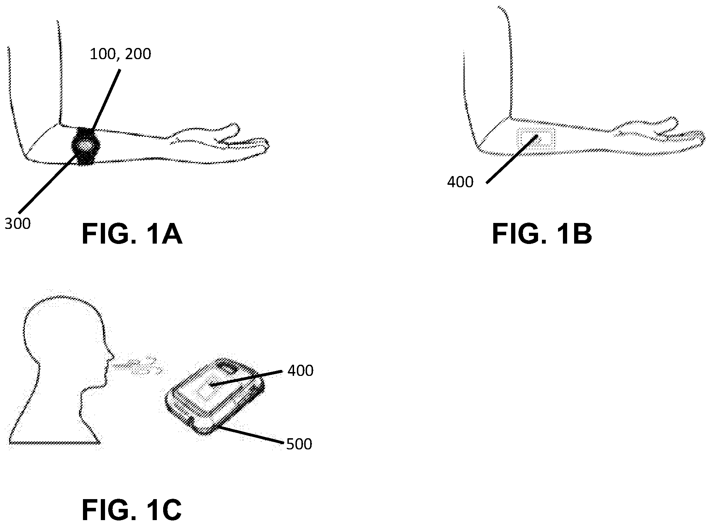

**FIG. 1A, FIG. 1B,** and **FIG. 1C** illustrate coupling of the systems and devices of the present disclosure to a user and a connected device, in accordance with some embodiments. Systems and devices of the present disclosure may be biosensing systems. The biosensing system may be configured to monitor various physiological signals, such as glucose or lactic acid levels. The biosensing system may monitor electrolyte levels of a user. The biosensing system may monitor these signals by receiving bodily fluid such as sweat, breath, or saliva. The systems or devices referred herein may refer to a SweatSmart^{®} system. The SweatSmart^{®} system may collectively also be referred to as a SweatSmart^{®} device. In some instances, the sensor may be disposable while the transmitter and the forearm attachment may not be.

In an aspect, a wearable sensing apparatus is provided. The wearable sensing apparatus may comprise a plurality of modular sensors configured to detect one or more biomarkers in a biological fluid sample of a subject when the device is being worn by the subject; and a device configured to interchangeably and releasably couple to a modular sensor selected from the plurality of modular sensors, wherein the device is configured to receive and store sensing signals from the modular sensor.

As illustrated in **FIG. 1A****,** a transmitter module **200** comprising a sensor **100** of the present disclosure may be integrated into a wearable attachment in the form of an armband **300.** The band can be designed to be worn around the forearm, side arm, lower back, legs, etc, using a mechanism such as a Velcro or a strap mechanism. The bottom layer of the band can be embedded with a wavy silicone pattern to avoid slipping while on skin. Pores can be embedded into the band for breathability. A cavity, e.g. a hole, can be provided in the band for inserting the transmitter module **200** from the bottom side of the band. Once the transmitter module **200** (with the sensor **100)** is inserted into the band and tightly fitted, the band can be strapped around the forearm or other desired area on the skin for continuous monitoring of metabolites including glucose and other markers from sweat.

As illustrated in **FIG. 1B****,** a transmitter module **200** comprising a sensor **100** of the present disclosure may be integrated into a wearable attachment in the form of a patch **400.** The patch **400** may comprise an adhesive layer coupled to a mount. The mount may be provided with a cavity with an opening towards the skin. The transmitter module **200** (with the sensor **100)** can be inserted onto the patch from the top side and fitted using mechanisms such as magnetic or mechanical mechanisms. For example, the transmitter module can be snapped onto the sensor substrate (e.g., of the patch) using a combination of magnetic and mechanical forces. The adhesive protective film can be peeled and then placed on the skin for continuous disease monitoring from sweat. While a patch configured to receive a transmitter is described, it is to be understood that the patch may comprise an integrated transmitter as described elsewhere herein. For example, the patch may be provided with a fully integrated transmitter that is not removable from the patch.

While biosensing systems utilizing a wrist strap form and a patch form are described herein, it is to be understood that the biosensing system may comprise any form. For example, the biosensing system may be integrated into an arm band, a head band, a leg strap, a chest strap, an ankle band, etc. The biosensing system may be integrated into an article of clothing, for example, within a compression fit garment, such as a sock, a shirt, a pants, a sleeve, etc. The biosensing system may be utilized to monitor the skin in proximity to the ankles, calf muscles, knees, quadriceps, hamstrings, hips, obliques, ribs, intercostal muscles, sternum, clavicle, pectorals, deltoids, shoulders, latissimus dorsi, biceps, triceps, elbows, forearms, or wrists.

In some cases, the biosensor of the present disclosure may not be connected physically connected to a user. For example in some cases, the biosensor may be a standalone device. For example, the biosensor in some cases may be attached to a connected device, as disclosed elsewhere herein. For example, a biosensor which may not be connected physically to a user may be a breath sensor. For example, a biosensor which may not be connected physically to a user may be used to analyze a biological fluid which is drawn from a user (e.g. blood, amniotic fluid, etc.) and placed on a sensor such as a modular sensor. A biosensor may be in proximity to a user. For example, a biosensor which may not be connected physically to a user may be an environmental sensor. In some cases, transmitter module **200** with sensor **100** may be a standalone device. In some cases, a patch system of the present disclosure may be used to attach a transmitter module **200** or an integrated transmitter module **200** to a connected device. In some cases, a strap as disclosed herein may be utilized to attach a transmitter and sensor to a connected device. In some cases, an attachment device of the present disclosure may be used sensor-side up in order to expose a sensor to an air-borne biological fluid, such as breath.

As illustrated in **FIG. 1C****,** a transmitter module **200** comprising a sensor of the present disclosure can be integrated with a patch **400** placed on the back of connected device **500,** such as a mobile phone for breath sensing applications. A breath sensing system of the present disclosure may be integrated into a watch, may be independent and have clip for attachment onto a garment, may be worn on a lanyard or chain, etc.

According to some aspects of the disclosure, a modular sensor (e.g. sensor **100)** is provided. The modular sensor may comprise a substrate; a plurality of contact electrodes provided on a surface of the substrate; and a plurality of sensing lines disposed between the plurality of contact electrodes to collectively form a plurality of sensor elements, wherein each sensor element comprises at least one sensing line extending longitudinally between a pair of contact electrodes, wherein the modular sensor is configured to be operably and releasably coupled to a device for use as a wearable sensing apparatus.

Disclosed herein is a modular sensing kit. The kit may comprise one or more of a wearable attachment, a sensor, and a transmitter module of the present disclosure. The modular sensing kit may comprise (1) the wearable device (e.g. an attachment mechanism) and (2) the plurality of discrete biological or chemical sensors (e.g. sensor **100)** of any embodiment disclosed herein. In some embodiments, a quick release mechanism provided on the wearable device allows different discrete sensors to be manually attached and detached from the wearable device without the use of tools.

In some embodiments, the plurality of discrete sensors (e.g. sensor **100)** are provided separately from the wearable device. In some embodiments, one or more of the discrete sensors is configured for a single use with the wearable device, and discarded after each use encounter by the subject. In some embodiments, one or more of the discrete sensors is configured for multiple uses with the wearable device, and capable of being recycled and reused in multiple use encounters by the subject. In some embodiments, the plurality of discrete sensors have different sensitivities to a same target analyte or different target analytes. In some embodiments, the plurality of discrete sensors comprises a first sensor and a second sensor both configured to detect a target analyte, wherein the first sensor has a higher sensitivity than the second sensor. In some embodiments, the first sensor is capable of detecting a substantially lower level or concentration of the target analyte compared to the second sensor.

### SENSOR

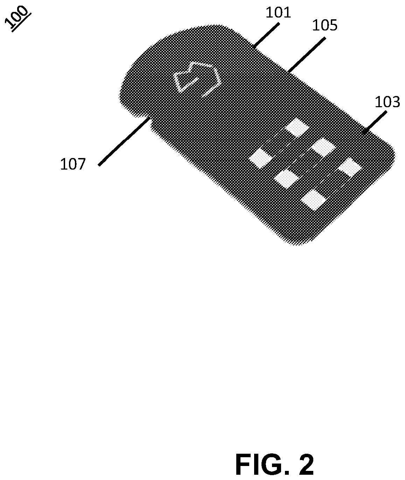

**FIG. 2****,** **FIG. 3A, FIG. 3B, FIG. 3C****,** **FIG. 4A, FIG. 4B, FIG. 4C****,** **FIG. 5A,** and **FIG. 5B** illustrate a sensor **100,** in accordance with some embodiments. In some embodiments, sensor **100** is a modular sensor. A modular sensor may be configured to function as an active sensing unit when electronically coupled to the device. In some embodiments, the modular sensor is configured to fit within a recessed housing on the device. In some embodiments, the modular sensor is protected by the recessed housing when the device is being worn by a subject. In some embodiments, the substrate comprises a ferrous metal or alloy, and the device comprises a magnetic material. In some embodiments, the modular sensor is configured to be coupled and held in place on the device via an attractive force between the magnetic material and the ferrous metal or alloy.

At least one of the plurality of sensing lines comprises a nanoscale material (e.g. graphene). In some embodiments, the plurality of sensing lines each comprises a nanoscale material (e.g. graphene). In some embodiments, the plurality of sensing lines may comprise graphene, carbon nanotubes, molybdenum sulfide, boron nitride, metal dichalcogenides, phosphorene, nanoparticles, quantum dots, fullerene, 2D nanoscale material, 3D nanoscale material, 0D nanoscale material, 1D nanoscale material, or any combination thereof.

In some embodiments, the plurality of sensor elements is configured to detect one or more biomarkers in a biological fluid sample of a subject when the device is being worn by the subject. In some embodiments, the plurality of sensor elements is configured to detect a same biomarker. In some embodiments, each of the plurality of sensor elements is configured to detect a different biomarker. In some embodiments, the biological fluid sample comprises sweat or breath. In some embodiments, the plurality of sensor elements is configured to detect the one or more biomarkers when in contact with the biological fluid sample.

In some embodiments, the plurality of sensor elements is capable of detecting the one or more biomarkers in a non-invasive manner, without requiring penetration of the subject's skin to extract the biological fluid sample. In some embodiments, the plurality of sensor elements is configured to detect a presence and concentration of the one or more biomarkers substantially in real-time when the device is being worn on the subject. In some embodiments, data indicative of the presence and concentrations of the one or more biomarkers is collected and stored by the device. In some embodiments, the data is collected and stored on the device over a time period that the device is being worn on the subject. In some embodiments, the modular sensor is configured to be operably and releasably coupled to the device without the use of tools. In some embodiments, the modular sensor is configured to be operably and releasably coupled to the device in less than 10 seconds.

The sensor may be removably coupled to the transmitter. The sensor a sensor substrate, electrodes, and sensor elements such as graphene. The weight of the biosensing system may be negligible, for example, equal to or less than about 500g, 400g, 300g, 200g, 150g, 120g, 100g, 80g, 60g, 40g, 30g, 20g, 10g, 5g, 4g, 3g, 2g, or 1g. Optionally, the sensor may be a disposable sensor. Alternatively or in addition, the sensor may be a replaceable sensor. For example, the sensor may be used, cleaned or processed, and be used again. While disposable sensors are primarily discussed herein, it is to be understood that details and/or descriptions discussed with respect to disposable sensors may be applicable to replaceable sensors.

**FIG. 2** shows an isometric view of a sensor **100,** in accordance with some embodiments. As described herein, the sensor **100** may be configured to be removably coupled to a transmitter module **200.** The sensor may comprise a sensor substrate **101,** electrode contacts **103,** sensor elements **105,** and a notch **107.** Optionally, the sensor elements may comprise graphene. The sensor may be a non-invasive sensor and can be utilized to screen bodily fluids for disease and micronutrient information. The sensor can comprise a bio-chemical sensor element functionalized to detect glucose, lactic acid, electrolytes, and/or other biomarkers from sweat, breath, saliva etc. The sensor can comprise a minimal footprint and a small form factor. For example, the sensor can comprise a volume equal to or less than about 50 cm³, 40 cm³, 30 cm³, 20 cm³, 18 cm³, 16 cm³, 14 cm³, 12 cm³, 10 cm³, 9 cm³, 8 cm³, 7 cm³, 6 cm³, 5 cm³, 4 cm³, 3 cm³, 2 cm³, 1 cm³, or any volume therebetween. Sensor **100** may be replaceable. Sensor **100** may be disposable. In some instances, the sensor may be disposable while the transmitter module **200** and the wearable attachment **300** may not be.

As shown, the sensor **100** may comprise a sensor substrate **101.** As disclosed herein, a substrate **101** can be polyamide, Polyethylene terephthalate (PET), polydimethylsiloxane (PDMS), Poly(methyl methacrylate) (PMMA), other plastics, silicon dioxide, silicon, glass, aluminum oxide, sapphire, germanium, gallium arsenide, indium phosphide, an alloy of silicon and germanium, fabrics, textiles, silk, paper, cellulose based materials, insulator, metal, semiconductor, or any combination thereof. The substrate can be rigid, flexible or any combination thereof. In some embodiments, the substrate can be a flexible substrate and the graphene layer can be deposited epitaxially, by exfoliation and deposition, etc.

As shown, the sensor **100** may comprise a notch **107.** The notch **107** may aid in the alignment of the sensor **100** with the transmitter module **200.** The notch on the sensor substrate may aid in alignment of the sensor as it is placed into the transmitter module. The notch may specify a direction in which the sensor may be attached to the transmitter module, e.g., the biosensor element facing outward. Such a design can prevent any damage to the sensor element.

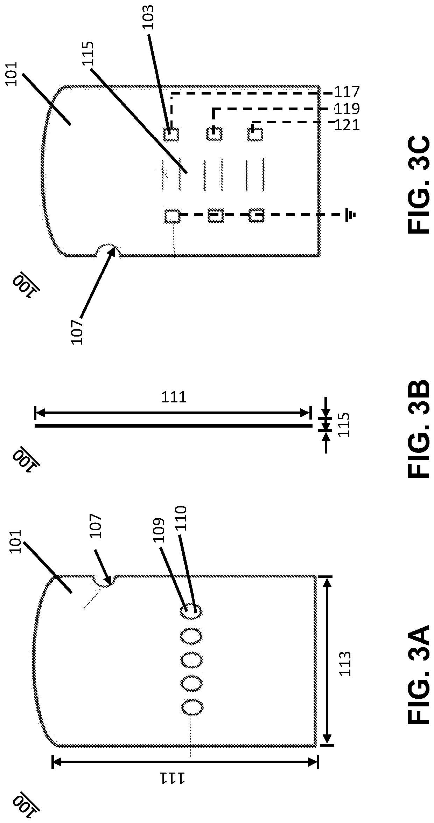

**FIG. 3A** illustrates a top view of a sensor **100,** in accordance with some embodiments. As illustrated, the sensor may comprise electrodes **109** that allow coupling with a transmitter module **200** described herein. The electrodes may be embedded in the substrate. The electrodes may comprise metal contacts. For example, the electrodes of the sensor may be configured to couple to electrode contacts of a transmitter and allow electrical signals to be transmitted and/or received between the two. Metal contact areas can be embedded onto the back side of the substrate to allow for electrical connection to the transmitter module's sensor readout system. The surface area of the backside metal contacts can be fabricated larger than a pogo pin tip area on the transmitter module so as to ensure robust electrical connection between the sensor substrate and the transmitter.

The sensor may comprise one or more magnetic components. For example, the sensor substrate may itself be magnetic. In some examples, sensor substrates may comprise embedded magnetic elements such as an embedded ferrous material. The substrate may comprise embedded sensor electrodes with metal vias. For example, the electrodes **109** may be metal vias. The vias may be formed around iron or another ferrous material **110.** The ferrous material may be a ferrous core. The vias may be formed around an iron sheet. Magnetic sensor substrates with embedded metal contacts can be formed by folding and adhesively bonding flexible printed circuits (FPC) substrates around thin iron sheets.

In some examples, the sensor may be coupled to the transmitter using one or more of the following attachment mechanisms: clips, latches, snaps, straps, tethers, tape, Velcro^{™}, hook and loop, tack features, screw fasteners, tabs, magnetic fasteners, or any other suitable connection mechanisms such as elastic bands and adhesives.

The thickness, surface area and overall design of the substrate can be such that it accommodates for ease of user handling. In an example there may be additional contact surface area for picking up with fingers such that it may possible to pick up the sensor without contacting the biochemical sensor element area. The sensor may comprise a first dimension **111** and a second dimension **113.** The first dimension may be between 25 millimeters (mm) and 35 mm and the second dimension may be between 15 mm and 20 mm. The first dimension may be between 10 millimeters (mm) and 50 mm and the second dimension may be between 5 mm and 40 mm. The first dimension may be between 3 millimeters (mm) and 60 mm and the second dimension may be between 1 mm and 30 mm.

**FIG. 3B** illustrates a side view of a sensor **100,** in accordance with some embodiments. Sensor **100** may comprise a first dimension **111** and a third dimension **115.** The first dimension may be between 30 mm and 35 mm and the third dimension may be less than 1 mm. The first dimension may be between 10 mm and 50 mm and the third dimension may be less than 5 mm.

**FIG. 3C** illustrates a bottom view of a sensor **100,** in accordance with some embodiments. The bottom of the sensor **100** may be configured to contact a biological fluid of the present disclosure. As shown, sensor **100** comprises substrate **101,** electrode contacts **103,** and sensor element deposition areas **115.** Sensor elements **105** may be deposited on sensor deposition areas **115.** Sensor elements **105** may comprise embodiments, variations, and examples of a bio-sensor element as disclosed herein. Sensor elements **105** may comprise graphene.

Electrode contacts **103** may allow electrical coupling between sensor elements **105** and electrodes **109,** which then may allow electrical contact to transmitter **200.** The electrode contacts **103** may be embedded in the substrate. The electrode contacts **103** may be metal contacts. For example, the electrode contacts **103** of the sensor may be sufficiently conductive to couple to electrodes **109** and allow signals to be transmitted and/or received between the two. Metal contact areas can be embedded onto the substrate to allow for electrical connection to the transmitter module's sensor readout system. As shown the electrode contacts one side each sensor element may be connected to a common ground. The common ground may be a virtual ground. As shown each sensor element may be connected in parallel to individual electrodes **109** on the opposite side of the sensor. This arrangement may allow for each sensor element to be read in parallel. For example, each sensor element may be multiplexed. For example, three electrode contacts on a sensor **100** may comprise electrical leads **117, 119,** and **121,** which may each connect to three electrodes **109** on an opposing surface of the sensor. The ground may connect to a ground electrode on an opposing surface of the sensor. Each sensor element may in this fashion be connected via pogo pins to the analog to digital converter within a transmitter module **200.**

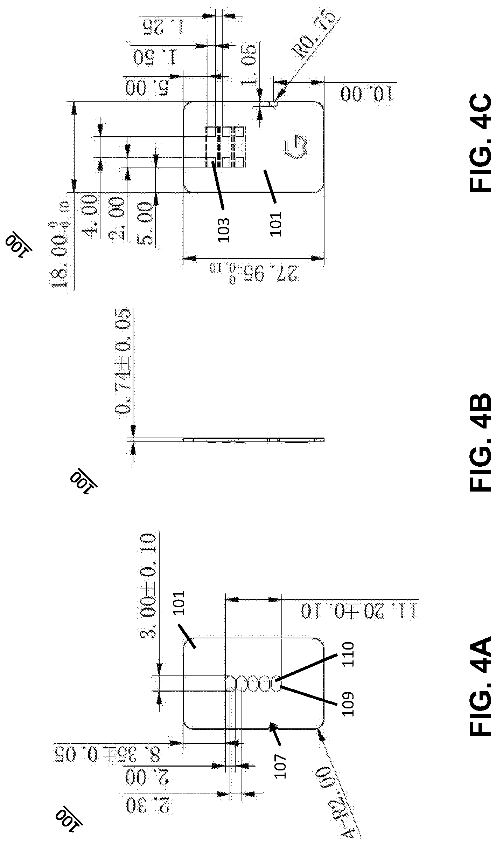

**FIG. 4A, FIG. 4B,** and **FIG. 4C** illustrate an additional example of a sensor, in accordance with some embodiments. As shown in **FIG. 4A****,** the electrodes **109** on the transmitter module facing side of the sensor may aligned perpendicularly to the long axis of the device. The electrode contacts and electrodes may be arranged in varying patterns and angles. As shown in **FIG. 4A****,** the sensor **100** may be made smaller or larger, may be any shape, and may be of any aspect ratio. The sensor may be sufficient size that it may be handled by a user without touching the sensor elements **105.**

### BIO-SENSOR METAL CONTACTS

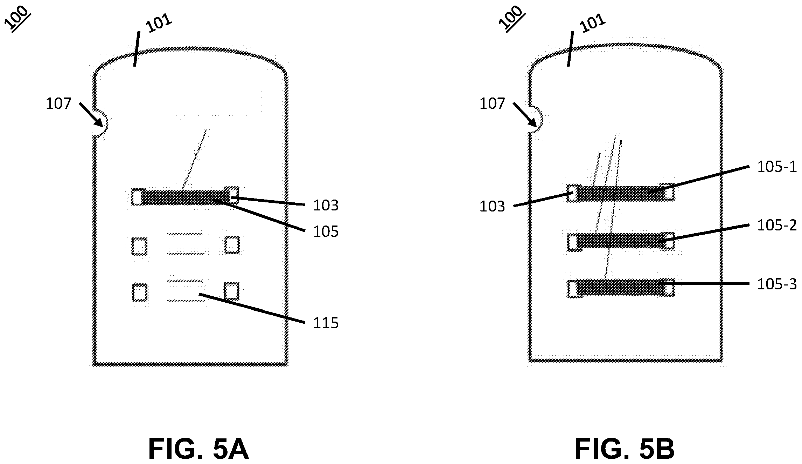

**FIG. 5A** and **FIG. 5B** illustrate an example of a sensor substrate **101** with a single sensor element **105** and an example of a sensor substrate **101** with multiplexed sensor elements **105-1, 105-2,** and **105-3,** in accordance with some embodiments. In some cases, the sensor elements may be multiplexed. In some embodiments, there may be two or more sensor elements. In some embodiments, there may be less than 100 sensor elements. For example, there may be 2, 3, 4, 5, 6, 7, 8, 9, 10, 12, 15, 20, 25, 30, 50, 100, 1000, or more sensor elements. In some embodiments, the different multiplexed configurations permit a plurality of different target analytes to be detected from a sample of the subject collected on the wearable device when the subject is wearing the device. For example, the three or more discrete sensors comprises a first sensor for detecting a first target analyte, a second sensor for detecting a second target analyte, and a third sensor for detecting a third target analyte. Each sensor element may be configured to detect a particular target analyte. The sensor may detect two or more of electrolytes, glucose, lactic acid, IL6, a cytokine, HER2, Cortisol, ZAG, cholesterol, vitamins, ions, a protein, a drug molecule, a metabolite, a peptide, an amino acid, a DNA, an RNA, an aptamer, an enzyme, a biomolecule, a chemical molecule, or a synthetic molecule. The sensor may detect electrolytes, glucose, and lactic acid.

In some embodiments, the different multiplexed configurations enable increased sensitivity in the detection and monitoring of different target analytes. For example, the wearable device may comprise a processing module operably coupled to three or more different discrete biological or chemical sensors. The processing module may be configured to selectively activate the three or more different discrete biological or chemical sensors in different multiplexed configurations depending on desired type(s) of sensing application of a subject. For example, the processing module may be configured to selectively activate a fewer number of the biological or chemical sensors to reduce power consumption of the wearable device. Additionally or alternatively, the processing module may be configured to selectively activate a greater number of the biological or chemical sensors to enhance sensitivity in the detection and monitoring of different target analytes.

In some embodiments, the processing module is configured to selectively activate at least two out of the first, second and third sensors. In some embodiments, the processing module is configured to selectively activate (1) the first and second sensors in a first multiplexed configuration, (2) the second and third sensors in a second multiplexed configuration, or (3) the first and third sensors in a third multiplexed configuration. In some embodiments, the processing module is capable of detecting (1) the presence and (2) concentrations ranging from femtogram/liter 1 fg/L to 1,000 ng/L of two or more different target analytes in a sample having a volume of less than 100 µL collected from the subject on the wearable device when the subject is wearing the device. The processing module may be capable of detecting concentrations greater 1 femtogram/liter (fg/L). The processing module may be capable of detecting concentrations greater 250 femtogram/liter (fg/L). The processing module may be capable of detecting concentrations greater than 0.250 ng/L in a sample. The processing module may be capable of detecting concentrations greater than 0.100 ng/L in a sample. The processing module may be capable of detecting concentrations greater than 0.250 ng/L in a sample having a volume of less than 10 µL. The processing module may be capable of detecting concentrations greater than 1 fg/L in a sample having a volume of less than 1 µL. The processing module may be capable of detecting concentrations greater than 250 fg/L in a sample having a volume of less than 1 µL. The processing module may be capable of detecting concentrations greater than 0.250 ng/L in a sample having a volume of less than 1 µL. The processing module may be capable of detecting concentrations greater than 250 fg/L in a sample having a volume of less than 0.1 µL.

In some embodiments, the wearable device is capable of detecting the presence and concentrations of the two or more different target analytes in less than 1 second. In some embodiments, the wearable device is capable of detecting the presence and concentrations of the two or more different target analytes in less than 100 milliseconds. In some embodiments, the wearable device is capable of detecting the presence and concentrations of the two or more different target analytes in less than 1 millisecond.

As shown, the sensor element (e.g., graphene or any nanoscale material layer) may be deposited on a sensor substrate. A sensor element of the present disclosure may comprise a field effect transistor comprising a drain electrode; a source electrode; an electrically insulating substrate; a nanoscale material layer arranged on the substrate; and a polar fluid induced gate terminal created by a polar fluid exposed to the nanoscale material layer. In some cases, the nanoscale material layer may partially define an electrically conducting and chemically sensitive channel. The nanoscale material layer and the channel may extend between and be electrically connected to the drain electrode and source electrode. In some embodiments, the polar fluid comprises the target analyte. In some embodiments, the polar fluid has a charge concentration sufficient to induce a polar fluid gate voltage that optimizes the gate voltage versus channel current characteristics of the field effect transistor in response to the target analyte.

A field effect transistor of the present disclosure may comprise a nanoscale material layer. The nanoscale material layer may comprise graphene, carbon nanotubes, molybdenum sulfide, boron nitride, metal dichalcogenides, phosphorene, nanoparticles, quantum dots, fullerene, 2D nanoscale material, 3D nanoscale material, 0D nanoscale material, 1D nanoscale material, or any combination thereof. A field effect transistor of the present disclosure may be a graphene field effect transistor. Any applicable method can be applied to fabricate a graphene field effect transistor, including, for example, the information disclosed in International Patent Publication No. WO2015/164,552, which is hereby incorporated by reference in its entirety. Sensors of the present disclosure may comprise a gateless graphene field-effect transistor, such as those described in Published International Patent Application No. WO2017/216641, which is incorporated herein by reference in its entirety.

In an example method of depositing a sensor element, a graphene sensor electrode with a functional back-polymer can be created. The back-polymer material can be chosen to bond, fuse, or adhere to a target substrate, such a substrate of the present disclosure and/or a flexible printed circuit. The graphene/polymer composite can then bonded to a substrate **101** at a deposition area **115** between embedded metal contacts **103** by heating the substrate beyond the fusing temperature of the back polymer. Graphene sensors may be additionally deposited by adhesive, by mechanical fastener, by deposition followed by encapsulation, by heat curing, by gluing, etc.

In some cases, the sensor substrate with bonded graphene biosensors (e.g., sensor elements **105)** can then later functionalized with selective bio chemical molecules. For example, the graphene layer may be functionalized with a receptor layer deposited on the nanoscale material (e.g. graphene) layer. The receptor layer may comprise receptors targeting a target analyte. In some embodiments, the receptors may comprise pyrene boronic acid (PBA), pyrene N-hydroxysuccinimide ester (Pyrene-NHS), organic chemicals, aromatic molecules, cyclic molecules, enzymes, proteins, antibodies, viruses, single stranded DNAs (ssDNAs), aptamers, inorganic materials, synthetic molecules, biological molecules. In some embodiments, the target analyte comprises an electrolyte, glucose, lactic acid, IL6, a cytokine, HER2, Cortisol, ZAG, cholesterol, vitamins, a protein, a drug molecule, a metabolite, a peptides, an amino acid, a DNA, an RNA, an aptamer, an enzyme, a biomolecule, a chemical molecule, a synthetic molecule, or combinations thereof.

For example, biosensors functionalized with PBA may exhibit a high sensitivity for D-glucose with a limit of detection (LOD) of 250 femtogram/liter. Pyrene Boronic Acid bonds to the Graphene surface using pi-pi bond. PBA forms a reversible boron-anion complex with D-glucose.

For example, biosensors may be functionalized with Lactate Oxidase (LOx) to the Graphene surface using an intermediate pyrene-NHS linking chemistry. The Lactate Oxidase may specifically bind to lactic acid molecules in the fluid. Biosensors functionalized with LOx may have a highly selective response (>94%) to Lactic concentrations in different control fluids. In another example, biosensors functionalized with Pyrene-NHS exhibited a high sensitivity for lactic acid with a limit of detection (LOD) of approximately 250 femtogram/liter; i.e., 2.78e-12 millimolar (mmol/L).

Without being limited by theory, the hydrophobicity of the graphene layer may lead to induced motion of the polar fluid over surface of biosensor. Increased hydrophobicity between graphene surface and the polar fluid may repel the polar fluid (like NaCl in DI water) from the graphene surface. The higher the concentration of the polar molecules (e.g., NaCl) in the fluid, the more the repelling effect may occur. This effect may be used to functionalize the surface of the sensor substrate. For example, a portion of the sensor substrate around the sensor deposition area may be functionalized with a hydrophilic material. The hydrophilic material may attract the biological fluid, e.g. sweat, toward the sensor element. A second portion of the sensor substrate near the sensor element may be not functionalized or may be functionalized with a hydrophobic material. The combination of these two functionalized portions may facilitate sweat localization on the sensor element and flow away from the sensor element.

Contacting graphene sensors to a sensory readout system can be challenging. One challenge in a commercial graphene sensor can be to connect the graphene sensor element to the outside world. In an example, metal contacts may need to be created on a graphene surface in order to connect a graphene sensor **105** to other elements. Metallization techniques can include creating metal contacts on a graphene surface using lithographic techniques including photolithography, e-beam lithography etc. However, certain metallization techniques can introduce defects, dopants, and irreversible contamination on the graphene sensors which could damage the material.

In some embodiments, the present disclosure provides methods to metallize graphene films to create electrical, optical or micro-electronic mechanical (MEMs) devices using printing techniques or dabbing/printing a liquid conduction paint. In an example method, skin safe conductive paint can be first dabbed onto either edges of the graphene sensor to form a source drain structure. The conductive paint can be dabbed, printed, painted, or applied. The conductive paint may be in limited contact or no contact with the electrode to prevent contact damage. The conductive ink can be printed, dabbed, painted or applied such that part of the ink is disposed onto the graphene surface of sensor element **105** and part onto a metal contact layer **103** underneath the graphene surface, which may be optionally pre-embedded into the sensor substrate **101.** The surface tension of the ink can allow it to spread over the metal contact surface underneath without leaking into the surrounding insulating flexible printed circuit area thus ensuring localization and limiting cross contamination.

In an example, the thickness of the ink can be in the range 40 microns to 500 microns. The thickness may be adjusted depending on the required ruggedization of the sensor element for example to prevent damage to the sensor while it was in contact with the skin. In some cases, the conduction ink thickness may be greater than 200 microns. In some cases, the conductive ink thickness may be less than 200 microns. In some cases, a passivation layer may be disposed on the ink. In some cases, the ink can be then cured by baking at an elevated temperature.

In some cases, a conductive ink may be composed of a conductive material, and a binder. In an example, a binder may be a resin or adhesive blended with a conductive material such as gold, silver, copper, nickel or other metals or alloys. In an example, a conductive material may comprise a conductive particulate such as a metal particulate. The conductive material may comprise conductive metal nanoparticles (NPs), alloys of the conductive metals, core/shell systems, etc. A metal particulate may have an average size of from about 0.5 to about 10 microns and as aspect ratio of at least about 3 to 1.

A resin or adhesive may be blended with a conductive material. In some examples, a polymer thick film may be formed. A resin or adhesive may be thermosetting, such as an epoxy, an acrylic, a polyester, etc. The polymer may be a thermoplastic polymer, such as, polyimide siloxane, nylon, neoprene, rubber, polyvinylbutyral terpolymer, etc. The binder and the conductive material may be dissolved in a solvent, such as a glycol ether or similar high vapor pressure solvent configured to evaporate over time.

Once the ink is cured, a passivation polymer can be dabbed/ printed over the metal contact layer such that the disposition area of passivation polymer was more than the metal contact layer area to avoid any exposure of the metal edges. The thickness of the passivation polymer layer can be chosen so as to ensure a final thickness of the metal passivation layer to be at least 200 microns. Such a thickness may limit wear or damage to the graphene sensor element while the sensor is in contact with the skin.

Optionally, specific non-bleeding materials can be chosen as passivation polymers, e.g., Acrylic, PMMA, silicone, polysilicone, PDMS, rubber, hotmelt co-polymers, EVA co polymers, ethylene acrylate, PET, Polyamide, PTFE, fluoropolymer, thermoplastics, gels, hydrogels, polypropylene, polyethylene, Polyolefins, polyvinyl chloride, polyesters, polyurethanes, Styrene block copolymers, Polycaprolactone, Polycarbonates, Fluoropolymers, Silicone rubbers, Thermoplastic elastomers, Polypyrrole, etc. The passivation layer may be polyurethane.

In some examples, more than one passivation layer may be applied. In some examples, a different passivation layer may be applied to the nanoscale material than is applied to the metalized region or the contact region. In some cases, the passivation layer is polyurethane and PMMA. The passivation layer may additionally aid attachment of the graphene layer.

Also disclosed is a method of fabricating a modular sensor. The method may comprise: (a) providing a sensor substrate comprising at least two electrodes disposed on a surface of the substrate; (b) depositing a layer of graphene on the surface of the sensor substrate between the at least two electrodes; (c) metallizing at least a portion of the layer of graphene at or near the at least two electrodes; passivating at least a portion of the layer of graphene with a passivation polymer; and (d) optionally, functionalizing at least a portion of the layer of graphene, wherein functionalizing the layer of graphene with a receptor layer, wherein the receptor layer is sensitive to a target analyte. The order in which some or all of operations (a) - (d) may be executed should not be deemed limiting. Rather, one of ordinary skill in the art having the benefit of the present disclosure will understand that some of (a) - (d) may be executed in a variety of orders not disclosed, or even in parallel. Some operations may be omitted.

In some embodiments, the depositing the graphene layer comprises heating the substrate beyond a fusing temperature of a functional back polymer disposed between the graphene layer and the substrate. In some embodiments, the method further comprises functionalizing a first portion of the substrate near the graphene layer with a hydrophilic material. In some embodiments, a second portion of the substrate near the graphene layer is not functionalized with the hydrophilic material. In some embodiments, the second portion of the substrate is functionalized with a hydrophobic material.

In some examples, a current is applied and a voltage is measured for each sensor element. In some examples, a voltage is applied and a current is measured for each sensor element. In some examples, an oscillating current or voltage is applied and a shift in the response frequency is measured. In some examples, a constant current of 5 to 200 micro Amps may be applied across sensor **105.** A time varying voltage from the sensor may be measured at the transmitter **200** which may be in electrical connection with sensor elements **105.** A voltage across sensor **105** may be readout 8 bit or higher by a DAC on board the transmitter module **200.**

In some embodiments, the transmitter module **200** may recognize sensor **100.** The transmitter may recognize the sensor as having been reused. The transmitter may recognize the sensor as not being manufactured by an approved source. The transmitter may recognize the sensor electronically, such as by way of a "handshake". The transmitter may recognize the sensor by radiofrequency identification tag. The transmitter may recognize the sensor by physical indicia such as a pattern on the surface of the sensor.

### TRANSMITTER/PROCESSING MODULE

In some embodiments, a wearable device may comprise a processing module configured to operably couple to at least one sensor selected from a group consisting of a plurality of discrete biological or chemical sensors, wherein two or more different sensors for detecting two or more different target analytes are interchangeably and releasably attachable to the wearable device, depending on a type(s) of target analytes to be detected from a sample of a subject collected on the wearable device when the subject is wearing the device. The processing module may comprise an embodiment, variation, or example of a transmitter module disclosed herein.

In some embodiments, the processing module is configured to detect and monitor levels of a first target analyte when a first sensor specific to the first target analyte is attached to the wearable device. In some embodiments, the processing module is configured to switch to detection and monitoring of a second target analyte when the first sensor is detached from the wearable device and replaced by a second sensor specific to the second target analyte. In some embodiments, the processing module is located onboard the wearable device, and configured to process sensor data substantially in real-time as the data is being collected by the at least one sensor, in order to detect and monitor levels of one or more target analytes. In some embodiments, the wearable device comprises a graphical display for displaying the detected levels of the one or more target analytes. In some embodiments, the processing module is configured to transmit the processed sensor data to a remote device, server or third party entity. In some embodiments, the processing module comprises a recommendation engine configured to prescribe certain corrective or mitigative actions to the subject, based on the detected levels of the one or more target analytes.

In some embodiments, the device comprises a transmitter configured to transmit the sensing signals over a network. In some embodiments, the transmitter is configured to transmit the sensing signals to a mobile device that is associated with and in proximity to the subject. In some embodiments, the device comprises a recessed housing configured to receive and support the modular sensor. In some embodiments, the device is releasably coupled to the modular sensor via a magnetic attachment mechanism. In some embodiments, the magnetic attachment mechanism comprises a magnetic material provided on at least one of the modular sensor and the device, and a ferrous metal or alloy provided on at least one of the modular sensor and the device. In some embodiments, the device is configured to be releasably coupled to a strap, wherein the strap is configured to be worn on a portion of the subject's body. In some embodiments, the plurality of modular sensors comprises at least one graphene-based sensor.

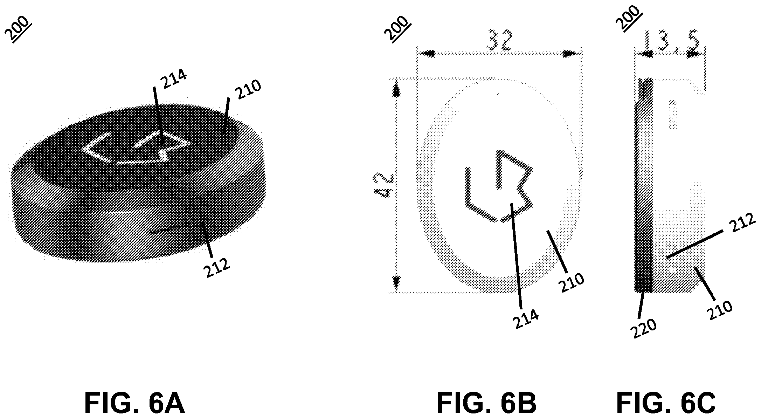

**FIG. 6A** illustrates a first example of a transmitter module **200,** in accordance with embodiments. The transmitter may comprise a processor, or a processing module. The transmitter module **200,** also referred to herein as a transmitter module or a processor module, may comprise an embedded sensor readout system. The transmitter module may have a top cover **210.** The top cover **210** may have indentations **212** which may aid in the attachment of transmitter module **200** to a wearable attachment of the present disclosure. The transmitter module **200** may have decorative elements on the top cover. In some cases, the top cover serves as a button **214.** In some cases, the button is a capacitive button. In some cases, the top cover is compressible to provide user input to the device.

**FIG. 6B** illustrates a top view of a transmitter module **200,** in accordance with some embodiments. As shown, the overall dimensions of the transmitter may be small. For example, the transmitter may comprise an overall volume equal to or less than about 500 cubic cm, 400 cubic cm, 300 cubic cm, 200 cubic cm, 150 cubic cm, 120 cubic cm, 100 cubic cm, 90 cubic cm, 80 cubic cm, 70 cubic cm, 60 cubic cm, 50 cubic cm, 40 cubic cm, 30 cubic cm, 20 cubic cm, 15 cubic cm, 10 cubic cm, 5 cubic cm, or any volume therebetween. While a transmitter having an oval shape is illustrated primarily herein, it is to be understood that the transmitter may comprise any shape, such as a circle, rectangle, etc.

As shown in **FIG. 6B****,** transmitter module **200** may comprise a first external dimension and second external dimension. The first external dimension may be 32 mm and the second external dimension may be 42 mm. The first external dimension may be within a range from 1 mm to 200 mm and the second external dimension may be within a range from 1mm to 200 mm. The first external dimension may be within a range from 5 mm to 50 mm and the second external dimension may be within a range from 10 mm to 100 mm. The transmitter may comprise a first exterior dimension **230,** a second exterior dimension **232,** and a third exterior dimension **234,** shown elsewhere herein.

**FIG. 6C** illustrates a side view of a transmitter module **200,** in accordance with some embodiments. The transmitter **200** may comprise a third external dimension. The third external dimension may be 13.5 mm. The third external dimension may be less than 100 mm. The third external dimension may be less than 30 mm. The third external dimension may be within a range from 1 to 100 mm.

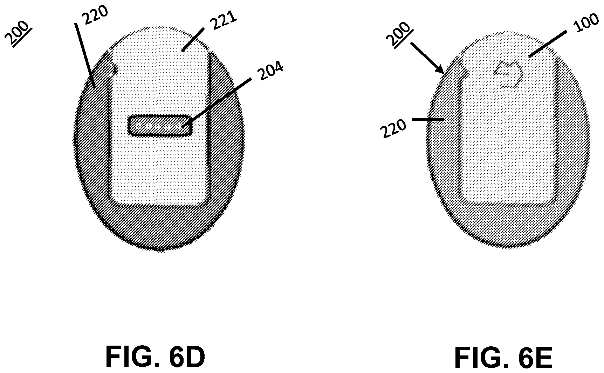

**FIG. 6D** illustrates a bottom view of a transmitter module **200** without a sensor **100** mounted thereon, in accordance with some embodiments. **FIG. 6E** illustrates a bottom view of a transmitter **200** with a sensor module **100** mounted thereon, in accordance with some embodiments. The transmitter module may accommodate multiple magnets that produced sufficient force to attract the replaceable sensor substrate when brought within sufficient proximity. In some instances, the transmitter may comprise a depression **221** on the bottom surface of the bottom cover **220** of the transmitter module. The depression may allow a sensor be attached such that a user facing surface of the sensor **100** is flush with the bottom surface of the bottom cover **220.** As shown, the transmitter may comprise various components, such as magnets, which allow easy coupling and uncoupling with a sensor. As shown, the transmitter module may comprise an electrical feed through area **204,** which may facilitate electrical contact between the sensor elements and a processing unit.

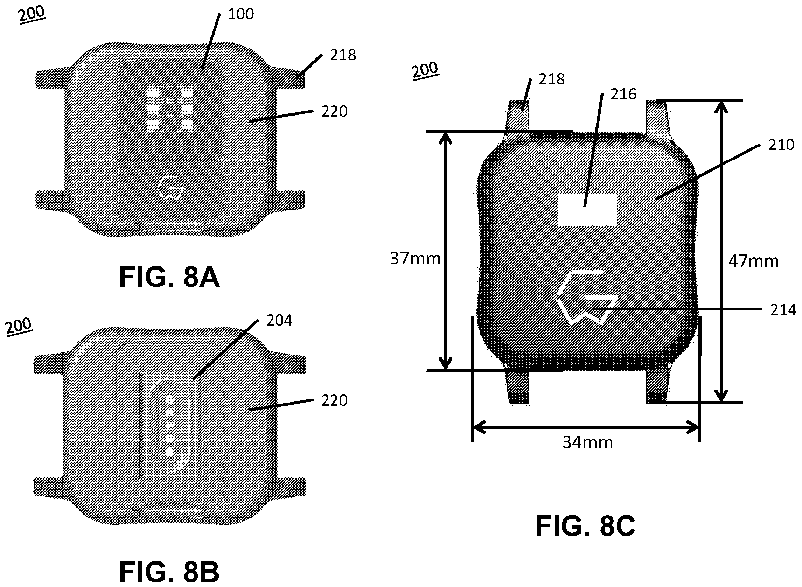

**FIG. 8A, FIG. 8B,** and **FIG. 8C** illustrate a second example transmitter module **200,** in accordance with some embodiments. **FIG. 8A** illustrates an example transmitter module in the form of a watch face, in accordance with some embodiments. Transmitter module **200** may be configured to couple with straps via strap mounts **218.** An example, embodiment, or variation of a sensor **100** of the present disclosure may be removably coupled the transmitter module **200.** The sensor **100** may be flush with a bottom surface **220** of the transmitter module **200.** In some cases the sensor may not be flush or, for example, may be slightly above the bottom surface **220,** which may aid in contact between the sensor elements and the skin.

**FIG. 8B** illustrates a bottom view of a second example of a transmitter module, in accordance with some embodiments. The transmitter module **200** may comprise an electrical feed through portion **204** on the bottom surface **200** of the transmitter module. The electrical feed through portion may comprise apertures for pogopins to pass through. As shown, the transmitter module may comprise an electrical feed through area **204,** which may facilitate electrical contact between the sensor elements and a processing unit. In some instances, the transmitter may comprise a depression on the bottom surface of the bottom cover **220** of the transmitter module.

**FIG. 8C** illustrates a top view of a second example of a transmitter module, in accordance with some embodiments. The size and scale of a second example of a transmitter module may substantially similar to other examples and embodiments described herein. The transmitter may comprise a first exterior dimension, a second exterior dimension, and a third exterior dimension, shown elsewhere herein. The first external dimension may be 34 mm and the second external dimension may be 37 mm. The first external dimension may be within a range from 1 mm to 200 mm and the second external dimension may be within a range from 1mm to 200 mm. The first external dimension may be within a range from 5 mm to 50 mm and the second external dimension may be within a range from 10 mm to 100 mm. The transmitter may comprise a third external dimension. The third external dimension may be 13.5 mm. The third external dimension may be less than 100 mm. The third external dimension may be less than 30 mm. The third external dimension may be within a range from 1 to 100 mm.

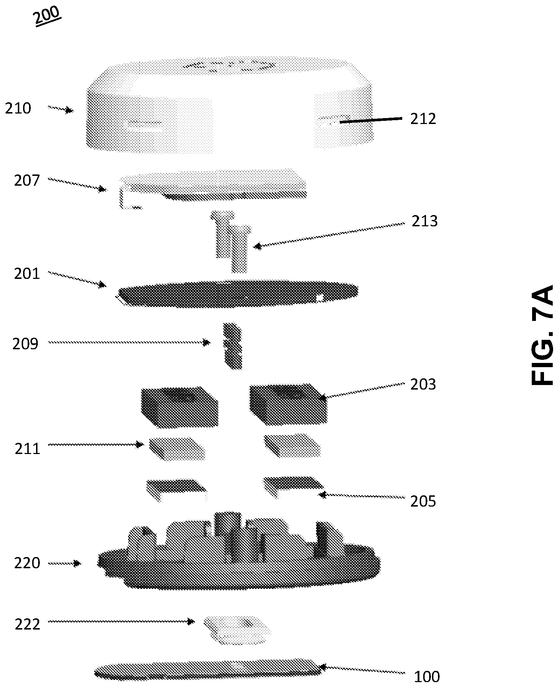

**FIG. 7A** illustrates an exploded view of the interior of a transmitter module **200,** in accordance with some embodiments. The transmitter may comprise a top cover **210** and bottom cover **220,** which together may house circuitry and other components which may used to power, control, or interface with a sensor **100** of the present disclosure. The top and bottom covers may be polycarbonate. The top and bottom covers may be plastic. The top and bottom covers may be colored or finished such that they are aesthetically pleasing. The transmitter **200** may comprise a dual battery scheme with power sharing mechanism, signal processing electronics, sensor interface electronics, ADC converters, integrated microprocessor unit (MCU) with embedded Bluetooth Low Energy (BLE) chip, antennae, and associated RF electronics.

The bottom cover **220** may comprise a seal ring **222** on an exterior surface, which may isolate electronics with the interior of the transmitter from biological fluids of the present disclosure. The seal ring may interface between an interior of the transmitter module and the exterior of the transmitter module via the feedthrough area **204.** The seal ring may be made of thermoplastic polyurethane. The transmitter may comprise one or more magnetic elements **205** in an interior of the transmitter. The one or more magnetic elements may be disposed on an interior surface of the transmitter **200.** The magnetic elements may be arranged to interface with a magnetic material on a sensor **100** of the present disclosure. The magnetic elements may comprise a ferrous material, such iron. The magnetic elements may comprise a permanent magnet. The magnetic material may comprise one or more of neodymium, samarium cobalt, alnico, ceramic, ferrite, iron, nickel, cobalt, or any other magnetic material. The magnetic material may be a permanent magnet. The magnetic material may be a rare Earth magnet. The magnetic element may be separated from one or more batteries by a spacer **211.** The spacer may be a shock absorber. The spacer may comprise ethylene vinyl acetate.

The interior of the transmitter may further comprise one or more batteries **203.** The batteries may have a charged voltage between 2 and 4.5 Volts. The batteries may carry a charge between 10 and 1000 milliamp hours. The batteries may carry a charge greater than 10 milliamp hours. The batteries may be lithium ion batteries. The batteries may be lithium ion polymer batteries.

As shown, transmitter **200** may comprise printed circuit board (PCB) **201.** PCB **201** may comprise a microcontroller unit with on board CPU, memory, data storage unit, three axis accelerometer, analog to digital converter (DAC), Bluetooth radio, etc. The PCB **201** may be attached to the bottom cover **220** by screws **213.** In some embodiments, the transmitter **200** may be disassembled and reassembled. In other embodiments, the transmitter **200** may not be disassembled without damage to one or more components, which may prevent tampering. While screws are provided in the illustrated embodiment, the device may be assembled using glues, solder, snaps, etc.

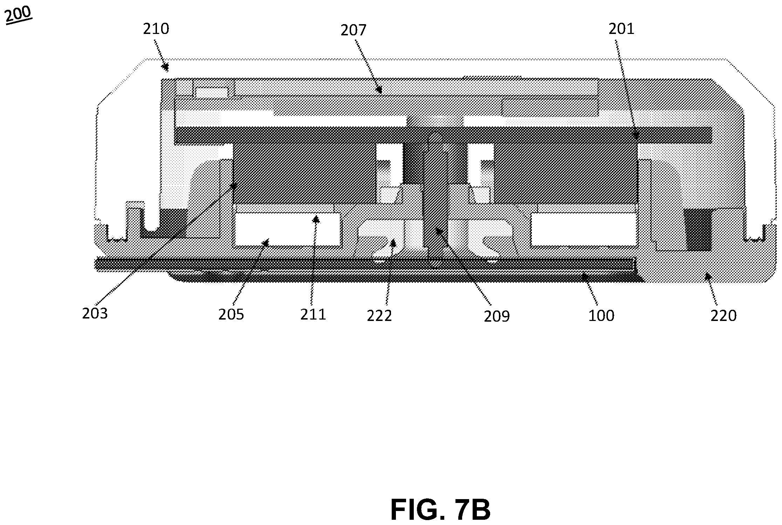

**FIG. 7B** illustrates a side view of an interior of a transmitter **200** along a slice through the transmitter, in accordance with some embodiments. As shown, the various components are arranged within the housing via compact design. As shown, the bottom cover **220** may comprise apertures within the feedthrough area **204** sized and shaped to receive pogo pins **209.** The pogo pins may be made of brass or another conductive material such as gold or copper. The pogo pins may be axially compressible. The pogo pins may comprise an internal spring to provide restoring force which may return the pogo pin to its original length after axial compression. Pogo pins may facilitate the absorption of excess kinetic energy of the sensor substrate as it snapped in. Pogo pins may facilitate consistent electrical connection between sensor **100** and printed circuit board **201.** Pogo pin may comprise a long work cycle to facilitate robust electrical connections between the sensor substrate and transmitter module. A pogo pin system may reduce any damage to transmitter module's sensor readout assembly. As shown, the pogo pins may be disposed between the batteries **203** to facilitate compact transmitter design.

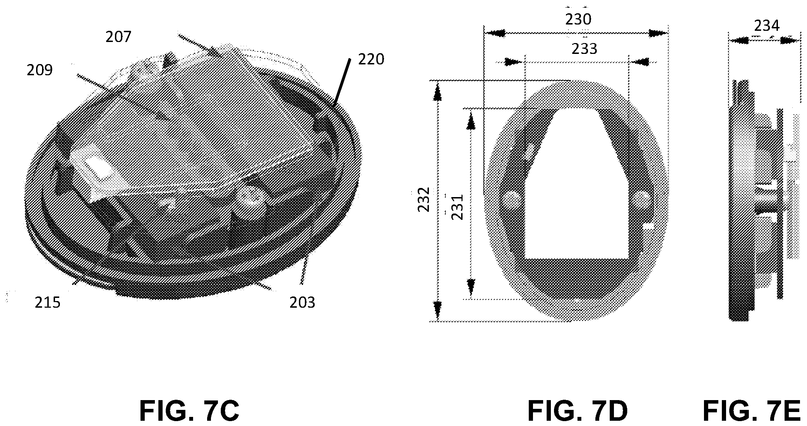

**FIG. 7C** illustrates an isometric view of a transmitter **200** with a top housing removed, in accordance with some embodiments. PCB **201** is removed for illustrative purposes. In some embodiments, the transmitter includes a display to send visual information to a user. The display may comprise one or more light emitting diodes (LEDs) **207.** The one or more LEDs may display light which may provide a visual cue to a user through the top cover **210.** The top cover **210** may comprise a transparent portion. In some examples, the transparent portion is rectangular. In some examples, the transparent portion is shaped like a logo. The one or more LED's may have one or more colors. For example, the LEDs may be blue, green, and red. The time duration and intensity of the LEDs may be varied to provide information to a user on the functioning or operation of the device. Multiple LEDs may be turned on at a single time in order to create a wide array of colors (e.g. a gamut). The LEDs may be electrically connected to PCB **201** via a serial AT attachment **215.**

**FIG. 7D** illustrates a top view of a transmitter **200** with a top housing removed, in accordance with some embodiments. **FIG. 7E** illustrates a side view of a transmitter **200** with top housing removed, in accordance with some embodiments. As illustrated, the transmitter **200** may comprise small dimensions. The transmitter may comprise a first exterior dimension **230,** a second exterior dimension **232,** and a third exterior dimension **234,** as described elsewhere herein. The transmitter may have first interior dimension **231** and second interior dimension **233.** The first interior dimension may be the length of PCB **201.** The second interior dimension may be the width of LED **207.** The first interior dimension may be between 30 mm and 35 mm, and the second interior dimension may be between 15 mm and 20 mm. The first interior dimension may be between 130 mm and 5 mm, and the second interior dimension may be between 115 mm and 2 mm. The first and second interior dimension may be less than the first and second exterior dimensions.

In some embodiments, PCB **201** includes a central processing unit (CPU, also "processor" and "computer processor" herein), which can be a single core or multi core processor, or a plurality of processors for parallel processing. PCB **201** also includes memory or memory location (e.g., random-access memory, read-only memory, flash memory). PCB **201** may include and electronic storage unit (e.g., hard disk or other non-volatile memory). PCB **201** may further comprise a communication interface disposed thereon (e.g., network adapter, a Bluetooth adapter, etc.) for communicating with one or more other systems, and peripheral devices. The memory, storage unit, communication interface and any other peripheral devices may be in communication with the CPU through a communication bus. The storage unit can be a data storage unit (or data repository) for storing data.

The transmitter **200** can be operatively coupled to a computer network ("network") with the aid of the communication interface. The communication interface may comprise or be in communication with an onboard Bluetooth radio. The MCU may comprise an RF antenna. The network can be the Internet, an internet and/or extranet, or an intranet and/or extranet that is in communication with the Internet. The network in some cases is a telecommunication and/or data network. The network can include one or more computer servers, which can enable distributed computing, such as cloud computing. The network, in some cases with the aid of the transmitter, can implement a peer-to-peer network, which may enable devices coupled to the device to behave as a client or a server.

In some embodiments, PCB **201** includes a digital to analog converter (DAC) disposed thereon. The DAC may convert analog data from the one or more sensor elements **105** to a digital signal. The DAC may receive a voltage from the one or more sensor elements. The DAC may digitize the voltage data with at least 8 bit resolution. The DAC may comprise one or more channels. In some cases, each sensor element may be connected to a single channel on a DAC. In some cases, each channel is read in sequence and digitized using a DAC with a single channel. In some cases, the digitized data may compressed, down sampled, or otherwise reduced in size in order to facilitate ease of transfer. The control and/or operation of the DAC may be operated by way of instructions from the CPU.

Data may be recorded continuously. In some cases, data may be recorded every 500 milliseconds (ms). In some cases, data may be recorded every 10 ms, every 20 ms, every 50 ms, every 100 ms, every 200 mx, every 500 ms, every second, ever 10 seconds, every minute, or more. Data may be recorded at interval within a range defined by any two of the preceding values. Data may be recorded for a period of time and then data collection may stop for a period of time. For instance, data may be collected for one minute, for one hour, for 2 hours, for 5 hours, for 1 day, for 5 days, for one year, or more. Data may be collected over a period of time within a range defined by any two of the preceding values. Data may be collected over a period time defined by one workout.

In some embodiments, PCB **201** includes a storage and/or memory device disposed thereon. The storage and/or memory device is one or more physical apparatuses used to store data or programs on a temporary or permanent basis. In some embodiments, the device is volatile memory and requires power to maintain stored information. In some embodiments, the device is non-volatile memory and retains stored information when the transmitter module is not powered. In further embodiments, the non-volatile memory comprises flash memory. In some embodiments, the non-volatile memory comprises dynamic random-access memory (DRAM). In some embodiments, the non-volatile memory comprises ferroelectric random access memory (FRAM). In some embodiments, the non-volatile memory comprises phase-change random access memory (PRAM). In further embodiments, the storage and/or memory device is a combination of devices such as those disclosed herein.

The CPU can execute a sequence of machine-readable instructions, which can be embodied in a program or software. The instructions may be stored in a memory location, such as the memory. The instructions can be directed to the CPU, which can subsequently program or otherwise configure the CPU to implement methods of the present disclosure. Examples of operations performed by the CPU can include fetch, decode, execute, and write back. The CPU can be part of a circuit, such as an integrated circuit. One or more other components disposed on PCB **201** can be included in the circuit. In some cases, the circuit is an application specific integrated circuit (ASIC) or a field programmable gate array (FPGA).

PCB **201** may comprise a storage unit. Storage unit can store files, such as drivers, libraries and saved programs. The storage unit can store user data, e.g., user preferences and user programs. The transmitter module **200** in some cases can include one or more additional data storage units that are external, such as located on a remote server that is in communication through an intranet or the Internet.

The transmitter module **200** can communicate with one or more remote computer systems through the network, for example, via a Bluetooth connection. For instance, the transmitter **200** can communicate with a remote computer system of a user. Examples of remote computer systems include personal computers (e.g., portable PC), slate or tablet PCs (e.g., Apple^{®} iPad, Samsung^{®} Galaxy Tab), telephones, Smart phones (e.g., Apple^{®} iPhone, Android-enabled device, Blackberry^{®}), or personal digital assistants.

PCB **201** may comprise a three axis accelerometer disposed thereon. For example, the accelerometer may be used to monitor motion of a user of the wearable device. A motion event detected by the accelerometer may turn on the device or start collection of data on the device. Lack of a motion event for a period of time may cause the device to enter a low power mode or turn off. Motion data may be logged with time to detect a correlation between the amount, type, or other quality of a biological fluid of the present disclosure with an amount or intensity of motion. The sensor data may be correlated with accelerometer data.

PCB **201** may comprise a battery charging integrated circuit disposed thereon. For example, the battery charging IC may monitor battery charging voltage. The battery charging IC may control battery charging voltage and/or current. For example, the battery charging IC may prevent over charging. The battery charging IC may increase battery life. Input power control may further be controlled through the use of one or more voltage regulators, such as a low drop out regulator.

PCB **201** may be in connection with and configured to receive data from an input device. In some embodiments, the transmitter module **200** may comprise an input device to receive information from a user. The input device may be a button on the exterior of the transmitter **200.** The button may turn on the device. The button may start or stop measurement. The button may reset the device. The button may be configured to start data or stop data sync to an external device. The MCU may be configured to receive information from the user in the form of one or more of: whether or not a button was pressed, the length of the press, and the number of sequential presses.

PCB **201** may be in connection with and configured to control a display disposed within the transmitter **200.** In some embodiments, the transmitter includes a display to send visual information to a user. The display may comprise one or more LEDs **207.** The one or more LEDs may display light which may visual to a user through the top cover **210.** The top cover **210** may comprise a transparent portion. In some examples, the transparent portion is rectangular. In some examples, the transparent portion is shaped like a logo. The one or more LED's may have one or more colors. For example, the LED's may be blue, green, and red. The time duration and intensity of the LEDs may be varied to provide information to a user on the functioning or operation of the device.

Reference is made to **Table 1** for example specifications of an MCU of the present disclosure. The following values are provided by way of example only and are not intended to be limiting.

**Table 1: Example MCU Specifications**

| **Technical Specifications** | |
|---|---|
| CPU frequency | 64MHz |
| ROM | 1MB |
| RAM | 256kB |
| Frequency Band | ±15KHz@2440MHz |
| Bluetooth | Bluetooth 5 |
| DCIN (Input voltage range) | DC 4.5V∼6V |
| DC power ripple | <50mV |
| Input current | <110mA |
| Programmable output power | -20dBm±3dBm~8dBm±3dBm |
| Receive sensitivity | <-90dBm |
| Charging time | <1.5h |
| LED | RGB back-light |

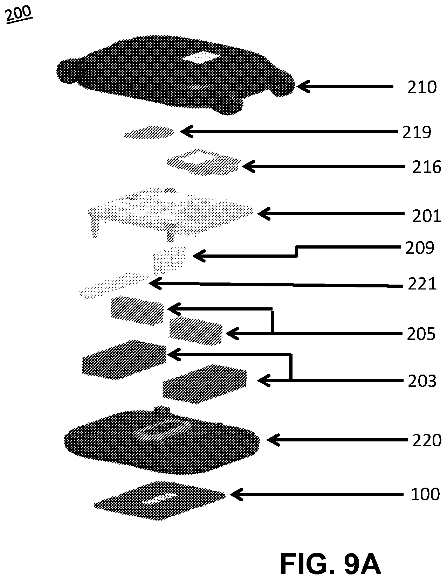

**FIG. 9A** illustrates an exploded view of the interior of a second example of a transmitter module **200,** in accordance with some embodiments. The transmitter may comprise a top cover **210** and bottom cover **220,** which together may house circuitry and other components which may used to power, control, or interface with a sensor **100** of the present disclosure. The top and bottom covers may be polycarbonate. The top and bottom covers may be plastic. The top and bottom covers may be colored or finished such that they are aesthetically pleasing. The transmitter **200** may comprise a dual battery scheme with power sharing mechanism, signal processing electronics, sensor interface electronics, ADC converters, integrated microprocessor unit (MCU) with embedded Bluetooth Low Energy (BLE) chip, antennae, and associated RF electronics, as described elsewhere herein.

The bottom cover **220** may comprise a seal ring on an exterior surface, which may isolate electronics with the interior of the transmitter from biological fluids of the present disclosure. The seal ring may interface between an interior of the transmitter module and the exterior of the transmitter module via the feedthrough area **204.** The seal ring may be made of thermoplastic polyurethane. The transmitter may comprise one or more magnetic elements **205** in an interior of the transmitter. The one or more magnetic elements may be disposed on an interior surface of the transmitter **200.** The magnetic elements may be arranged to interface with a magnetic material on a sensor **100** of the present disclosure. The magnetic elements may comprise a ferrous material, such iron. The magnetic elements may comprise a permanent magnet. The magnetic material may comprise one or more of neodymium, samarium cobalt, alnico, ceramic, ferrite, iron, nickel, cobalt, or any other magnetic material. The magnetic material may be a permanent magnet. The magnetic material may be a rare Earth magnet. The magnetic element may be separated from one or more batteries by a spacer. The spacer may be a shock absorber. The spacer may comprise ethylene vinyl acetate.

The interior of the transmitter may further comprise one or more batteries **203.** The batteries may have a charged voltage between 2 and 4.5 Volts. The batteries may carry a charge between 10 and 1000 milliamp hours. The batteries may carry a charge greater than 10 milliamp hours. The batteries may be lithium ion batteries. The batteries may be lithium ion polymer batteries.

As shown, transmitter **200** may comprise printed circuit board (PCB) **201.** PCB **201** may comprise a microcontroller unit with on board CPU, memory, data storage unit, three axis accelerometer, analog to digital converter (DAC), Bluetooth radio, etc. The PCB **201** may be attached to the bottom cover **220** by screws **213.** PCB 201 may be operatively coupled to a screen 219 may provide visual cues to a user. The screen may be an LCD. The screen may be an LED array. In some embodiments, the display is a cathode ray tube (CRT). In some embodiments, the display is a liquid crystal display (LCD). In further embodiments, the display is a thin film transistor liquid crystal display (TFT-LCD). In some embodiments, the display is an organic light emitting diode (OLED) display. In various further embodiments, on OLED display is a passive-matrix OLED (PMOLED) or active-matrix OLED (AMOLED) display. In some embodiments, the display is a plasma display. In other embodiments, the display is a video projector. In still further embodiments, the display is a combination of devices such as those disclosed herein.

In some embodiments, the transmitter **200** may be disassembled and reassembled. In other embodiments, the transmitter **200** may not be disassembled without damage to one or more components, which may prevent tampering. While screws are provided in the illustrated embodiment, the device may be assembled using glues, solder, snaps, etc.

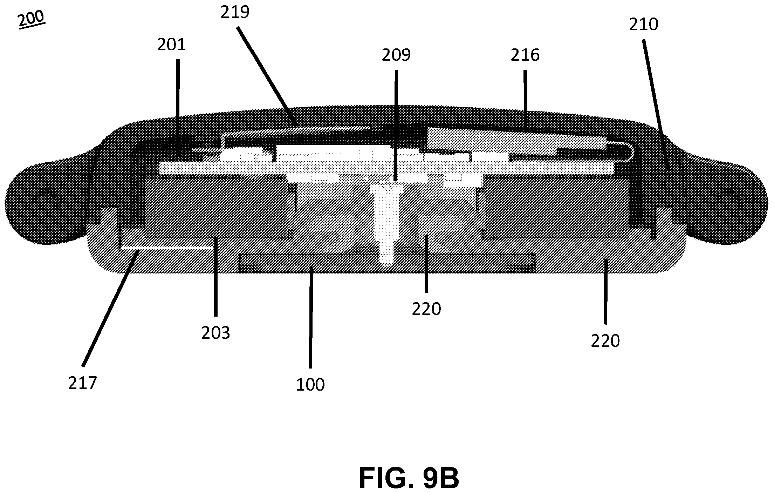

**FIG. 9B** illustrates a side view of an interior of a second example of a transmitter module **200** along a slice through the transmitter, in accordance with some embodiments. As shown, the various components are arranged within the housing via compact design. As shown, the bottom cover **220** may comprise apertures within the feedthrough area **204** sized and shaped to receive pogo pins **209.** The pogo pins may be made of brass or another conductive material such as gold or copper. The pogo pins may be axially compressible. The pogo pins may comprise an internal spring to provide restoring force which may return the pogo pin to its original length after axial compression. Pogo pins may facilitate the absorption of excess kinetic energy of the sensor substrate as it snapped in. Pogo pins may facilitate consistent electrical connection between sensor **100** and printed circuit board **201.** Pogo pin may comprise a long work cycle to facilitate robust electrical connections between the sensor substrate and transmitter module. A pogo pin system may reduce any damage to transmitter module's sensor readout assembly. As shown, the pogo pins may be disposed between the batteries **203** to facilitate compact transmitter design.

In some embodiments, the transmitter **200** may comprise user input mechanisms. The one or more user input mechanisms may comprise one or more buttons. For example the bottom cover **220** may comprise a button which is capacitive sensor **217.** For example, the top cover may comprise a second button which is a second capacitive sensor **219.** The one or more buttons may be conventional switches or mechanical buttons. The one or more buttons may be a keyboard. In some cases, the wearable device does not have a user input mechanism. In some cases, the wearable device may be controlled by a connected device, for example, a mobile device or a remote processor as described herein.

### USER INTERACTION

Methods as described herein can be implemented by way of machine (e.g., computer processor) executable code stored on an electronic storage location of the transmitter module **200** such as, for example, on the memory or electronic storage unit. The machine executable or machine readable code can be provided in the form of software. During use, the code can be executed by the processor. In some cases, the code can be retrieved from the storage unit and stored on the memory for ready access by the processor. In some situations, the electronic storage unit can be precluded, and machine-executable instructions are stored on memory.

The following are examples of user interactions with devices of the present disclosure and are not intended to be limiting. Other lighting schemes, including colors and durations, are envisaged. Other user initialed events are also contemplated.

Switch and LED light schemes
a. Turn on device: push button for three seconds confirmed with solid blue LED
b. Device connected: blue LED starts blinking every 5 seconds
c. Battery less than 10% charge: Magenta LED starts blinking every 5 seconds
d. Charging : < full charge - Magenta LED, full charge - green LED
e. Firmware update: push 8s, 3 color blinking in turn
f. When device comes into firmware update, if after 1 min, no update action, the device will turn off

Sensor light scheme:
a. Red for electrolytes, yellow for glucose, and white for lactic acid
b. If electrolytes, glucose, or lactic acid falls below a threshold: every 5 seconds blue blink and every minute respective electrolyte, glucose, and lactic acid colors flash in sequence quickly
c. If only one falls below threshold then only that respective color may flash.
d. If two fall then both will flash in an order that matches red first, yellow second and white last.

Sensor on/off light scheme
a. Sensor inserted: Device turns ON
b. If device is not connected: automatic turn off after 5 minutes
c. Automatic turn off when the sensor is removed

In some embodiments, the biosensor system has a screen. The screen may be on board a transmitter module 200. The screen may be associated with a connected device. In some cases, an onboard screen 216 may comprise provide values of a quantity of one or more of electrolytes, glucose, or lactic acid in, for example, milligrams per deciliter to a user. The values may be provided in substantially real time. The values may be time averages, rolling averages, maxima within a measurement period, minima within a measurement period, etc.

The transmitter may comprise a haptic feedback system. For example, the transmitter may vibrate and/or blink in order to prompt a user that a value has dropped below a threshold or raised above a threshold. For example, the transmitter may prompt a user to adjust a value of one or more of an electrolyte, glucose, or lactic acid. The transmitter may prompt a user to drink water. The transmitter may prompt a user to consume a dietary substance, such as sugar. The transmitter may prompt a user to temporarily discontinue physical activity, e.g. to rest. The transmitter may prompt a user to view data on a connected device.

### ARM BAND SYSTEMS

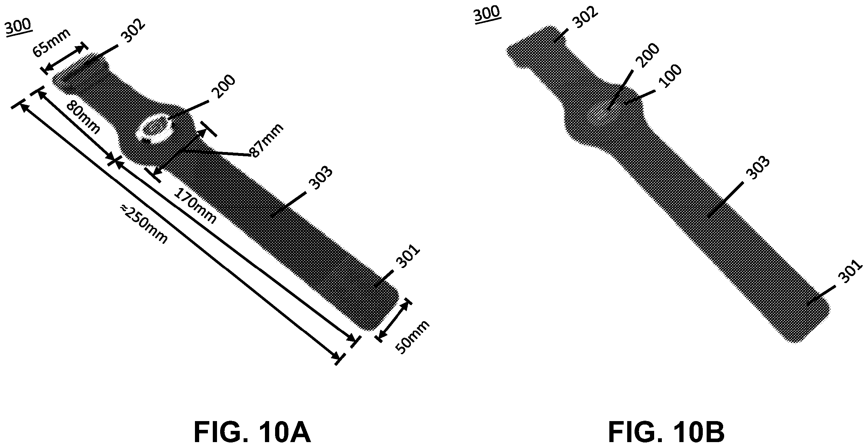

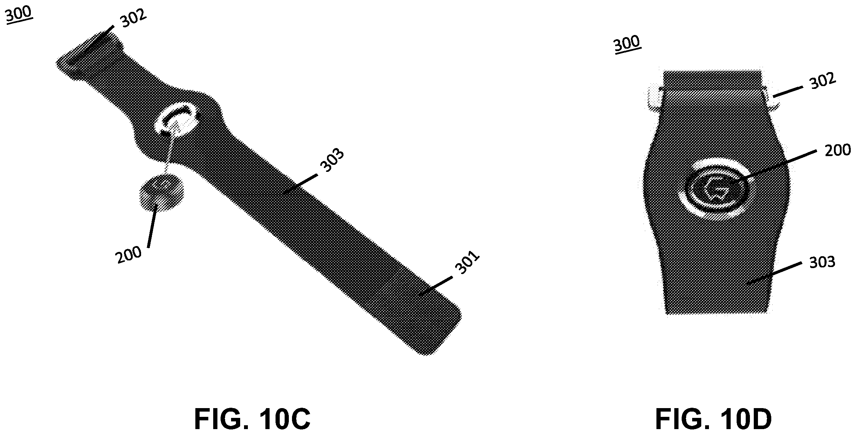

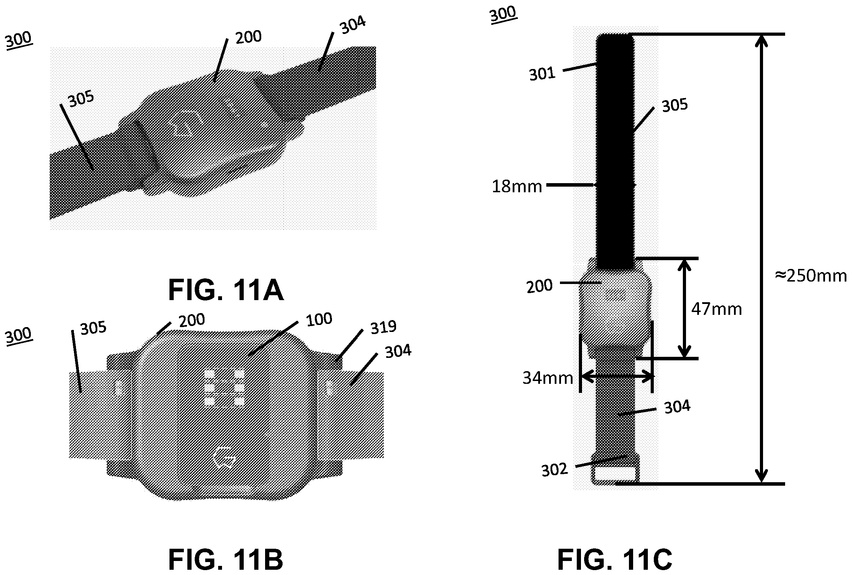

**FIG. 10A, FIG. 10B****,** **FIG. 10C, FIG. 10D****,** **FIG. 11A, FIG. 11B,** and **FIG. 11C** illustrate examples, embodiments, and variations of biosensor systems of the present disclosure. Such systems may comprise examples, embodiments, and variations of any of the transmitter modules **200** and sensors **100** described herein. A biosensor system of the present disclosure may be integrated into a wearable attachment. The wearable attachment may comprise an arm band. The wearable attachment may comprise a patch.

While biosensing systems utilizing a wrist strap form and a patch form are described herein, it is to be understood that the biosensing system may comprise any form. For example, the biosensing system may be integrated into an arm band, a head band, a leg strap, a chest strap, an ankle band, etc. The biosensing system may be integrated into an article of clothing, for example, within a compression fit garment, such as a sock, a shirt, a pants, a sleeve, etc. The biosensing system may be utilized to monitor the skin in proximity to the ankles, calf muscles, knees, quadriceps, hamstrings, hips, obliques, ribs, intercostal muscles, sternum, clavicle, pectorals, deltoids, shoulders, latissimus dorsi, biceps, triceps, elbows, forearms, or wrists.

**FIG. 10A** illustrates a transmitter module **200** comprising a sensor of the present disclosure integrated into a wearable arm band **300,** in accordance with some embodiments. The system may comprise a transmitter **200,** a strap **303,** and a sensor (not shown). As illustrated, the biosensing system may comprise small dimensions such that it can be worn discretely. For example, the biosensing system may be worn on a forearm, a wrist, or an upper arm of a user discretely. The strap **303** may comprise a buckle side **302** and a connecting side **301.** The strap **303** may comprise a natural or synthetic material. The material of a strap may be comfortable to a user. The material of a strap may be fabric, cloth, canvas, leather, cotton, nylon, polypropylene, polyester, flax, Lycra, Dyneema, Kevlar, Nomex, etc.

The bottom layer of the band can be embedded with a wavy silicone pattern to avoid slipping while on skin. Pores can be embedded into the band for breathability. A cavity, e.g. a hole, can be provided in the band for inserting the transmitter module **200** from the bottom side of the band. Once the transmitter module **200** (with the sensor **100)** is inserted into the band and tightly fitted, the band can be strapped around the forearm or other desired area on the skin for continuous monitoring of metabolites including glucose and other markers from sweat. In some cases, the transmitter module may be inserted into the band after tightening and provide an adequately tight fit for monitoring. For example, without loosening the band, the transmitter may be removed, the sensor may be changed, and the transmitter may be reinserted to continue monitoring.

The dimensions of an arm band system may be varied to fit the body part of a user being monitored. For example, the length of a band may be long enough to attach circumferentially around an arm, a leg, an ankle, a chest, etc. The band may be provided in varying lengths to fit various patient dimensions. The band may comprise sufficient band material securely attach via a buckling mechanism as described elsewhere herein. In an example, the total length of the band may be approximately 250mm. In an example, the total length of the band may be within a range from 100 mm to 1000 mm. In an example, the total length of the band may between 200 mm and 400 mm. The largest width of the arm band may be between 20 mm and 100 mm. The largest width of the band may be between 1 mm and 200 mm.

**FIG. 10B** illustrates a bottom view of a biosensing system, in accordance with some embodiments. As shown, the transmitter module **200** may house a sensor **100** which may be exposed on a bottom surface such that the sensor or a sensor element thereof may contact a user. The sensor element may come into contact with a biological fluid of a user as described elsewhere herein.

**FIG. 10C** illustrates a transmitter module **200** of the present disclosure being removable from the wearable arm band **100,** in accordance with some embodiments. As illustrated, the transmitter may be freely decoupled and coupled with the attachment. Optionally, the attachment may comprise a mating surface that may allow easy coupling and uncoupling with the transmitter. Optionally, the mating surface may be provided with magnets, hooks, notches, snap on mechanisms, etc that allow easy coupling and uncoupling with the transmitter. Optionally, the transmitter and the forearm attachment may be configured to be used for weeks, months, or years while the sensor may be configured to be used for a shorter period of time, e.g., hours, days, weeks, months, or years. The sensor **100** may be disposable. The sensor 100 may be replaceable.

**FIG. 10D** illustrates a wearable arm band with strap mechanism engaged, in accordance with some embodiments. While fabric hook and loop system with a buckle is shown, many possible clasping systems may be used. For example, the wearable arm band may have a watch buckle and free side with holes to receive the watch buckle. The wearable arm band may have a hook and loop fastener, such as Velcro. The fabric hooks may be on the buckle side and the fabric loops may be on the connecting side. As shown, the fabric of the strap may be partially hooks and partially loops on the connecting side such that when the connecting side is fed through a buckle on the buckling side the hooked portion and the looped portion may be in contact. The strap **303** may be adjustable. The strap **303** may comprise any system which holds a sensor of the present disclosure in contact with a skin of a user. The buckled may comprise an end fittings (e.g. S-hooks, snap hooks, bolt/anchor plates, J-hooks, flat hooks, etc.), a fasteners (e.g. over-center, cam, ratchet, etc.), or buckles (e.g. slide buckles, snap buckles, etc.). The user can mix and combine these individual components and place them in any desired positions on the apparatus and platform. The individual components can have various connection mechanisms such as: hook and loop (Velcro), snaps, tack features, screw fasteners, tabs, or any other suitable connection mechanisms such as elastic bands and adhesives.

**FIG. 11A, FIG. 11B,** and **FIG. 11C** illustrate a second example of a wearable arm band, in accordance with some embodiments. **FIG. 11A** illustrates a transmitter module **200** comprising a sensor of the present disclosure integrated into a wearable arm band **300,** in accordance with some embodiments. The system may comprise a transmitter **200,** a two part strap **304** and **305,** and a sensor (not shown). As illustrated, the biosensing system may comprise small dimensions such that it can be worn discretely. For example, the biosensing system may be worn on a forearm, a wrist, or an upper arm of a user discretely. The strap may comprise a first part **304** and a second part **305.** The first part and the second part of the strap may comprise a natural or synthetic material. The material of a strap may be comfortable to a user. The material of a strap may be fabric, cloth, canvas, leather, cotton, nylon, polypropylene, polyester, flax, Lycra, Dyneema, Kevlar, Nomex, etc.

The bottom layer of the band can be embedded with a wavy silicone pattern to avoid slipping while on skin. Pores can be embedded into the band for breathability. A cavity, e.g. a hole, can be provided in the band for inserting the transmitter module **200** from the bottom side of the band. Once the transmitter module **200** (with the sensor **100)** is inserted into the band and tightly fitted, the band can be strapped around the forearm or other desired area on the skin for continuous monitoring of metabolites including glucose and other markers from sweat. In some cases, the transmitter module may be inserted into the band after tightening and provide an adequately tight fit for monitoring. For example, without loosening the band, the transmitter may be removed, the sensor may be changed, and the transmitter may be reinserted to continue monitoring.

The first part and the second part may be removably couple to the transmitter module **200** from strap mounts **318.** Transmitter module **200** may be configured to couple with straps via strap mounts **218.** An example, embodiment, or variation of a sensor **100** of the present disclosure may be removably coupled the transmitter module **200.** As shown each of the first part and the second part of the strap have releases to decouple the straps from the transmitter module **200.**

**FIG. 11B** illustrates a bottom view of a biosensing system, in accordance with some embodiments. As shown, the transmitter module **200** may house a sensor **100** which may be exposed on a bottom surface such that the sensor or a sensor element thereof may contact a user. The sensor element may come into contact with a biological fluid of a user as described elsewhere herein. As illustrated, the strap parts may be freely decoupled and coupled with the transmitter module. Optionally, the transmitter module may comprise a mating surface that may allow easy coupling and uncoupling with the strap parts. Optionally, the mating surface may be provided with magnets, hooks, notches, snap on mechanisms, etc that allow easy coupling and uncoupling with the transmitter module. Optionally, the transmitter and the forearm attachment may be configured to be used for weeks, months, or years while the sensor may be configured to be used for a shorter period of time, e.g., hours, days, weeks, months, or years. The sensor **100** may be disposable. The sensor **100** may be replaceable.

**FIG. 11C** illustrates a top view of an arm band system with example dimensions, in accordance with some embodiments. The dimensions of an arm band system may be varied to fit the body part of a user being monitored. For example, the length of a band may be long enough to attach circumferentially around an arm, a leg, an ankle, a chest, etc. The band may be provided in varying lengths to fit various patient dimensions. The band may comprise sufficient band material securely attach via a buckling mechanism as described elsewhere herein. In an example, the total length of the band may be approximately 250mm. In an example, the total length of the band may be within a range from 100 mm to 1000 mm. In an example, the total length of the band may between 200 mm and 400 mm. The largest width of the arm band may be between 20 mm and 100 mm. The largest width of the band may be between 1 mm and 200 mm.

While fabric hook and loop system with a buckle is shown, many possible clasping systems may be used. For example, the wearable arm band may have a watch buckle and free side with holes to receive the watch buckle. The wearable arm band may have a hook and loop fastener, such as Velcro. The fabric hooks may be on the buckle side and the fabric loops may be on the connecting side. As shown, the fabric of the strap may be partially hooks and partially loops on the connecting side such that when the connecting side is fed through a buckle on the buckling side the hooked portion and the looped portion may be in contact. The strap parts together may be adjustable. The strap parts may comprise any system which holds a sensor of the present disclosure in contact with a skin of a user. The buckled may comprise an end fittings (e.g. S-hooks, snap hooks, bolt/anchor plates, J-hooks, flat hooks, etc.), a fasteners (e.g. over-center, cam, ratchet, etc.), or buckles (e.g. slide buckles, snap buckles, etc.). The user can mix and combine these individual components and place them in any desired positions on the apparatus and platform. The individual components can have various connection mechanisms such as: hook and loop (Velcro), snaps, tack features, screw fasteners, tabs, or any other suitable connection mechanisms such as elastic bands and adhesives.

### PATCH SYSTEMS

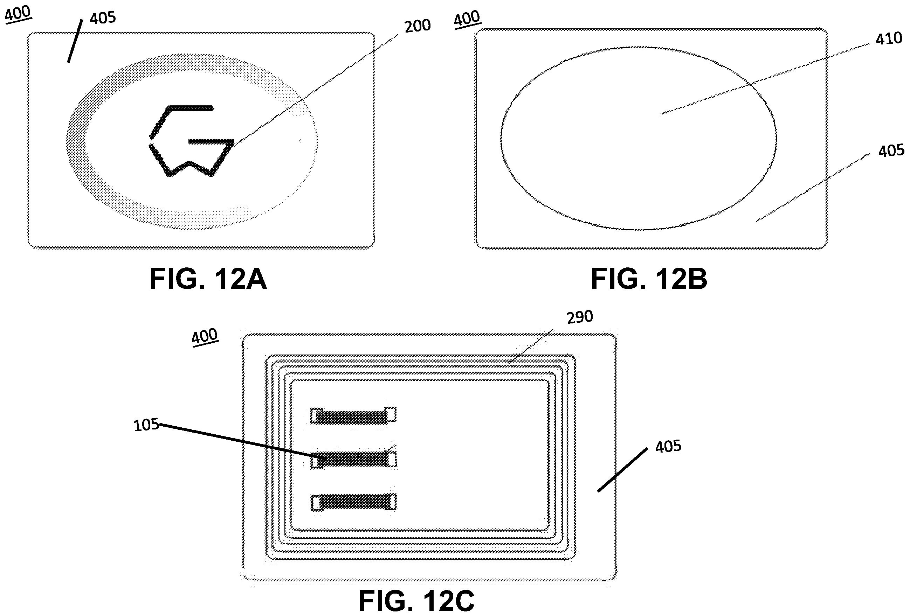

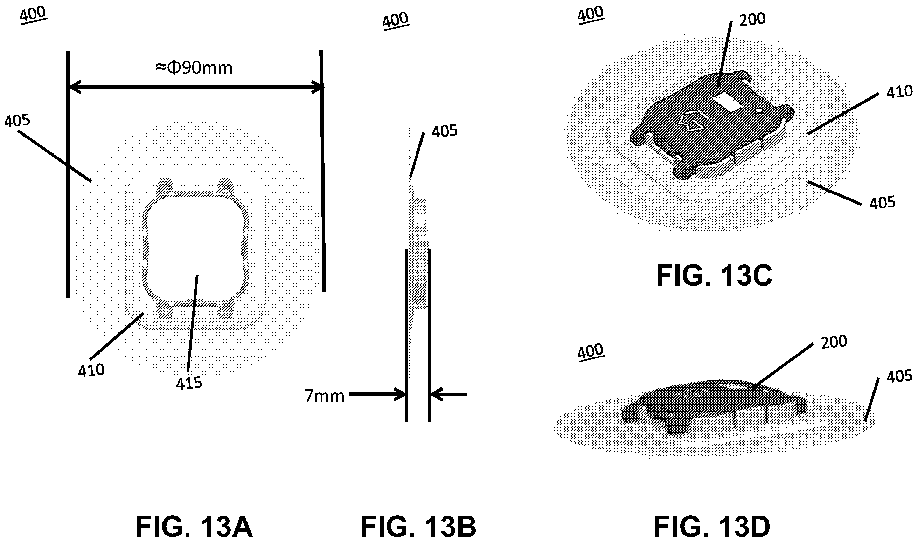

**FIG. 12A, FIG. 12B, FIG. 12C****,** **FIG. 13A,** and **FIG. 13B** show patch mounts **400** which may receive a transmitter module **200** of the present disclosure. A patch system of the present disclosure may comprise a receiving area and a planar portion **405** and a receiving portion **410.** Optionally in any of the embodiments disclosed herein, the planar portion can include an annular ring-like shape. An adhesive (not shown) can be placed on the planar portion of the housing base to promote adhesion of the device to the subject's skin, and to create a seal after the device has been placed onto the skin

**FIG. 12A** illustrates a top view of an example of a patch mount with a transmitter module mounted thereon, in accordance with some embodiments. **FIG. 12B** illustrates an example of a patch mount with a transmitter module decoupled from the patch mount, in accordance with some embodiments. Referring to **FIG. 12B****,** the planar portion **405** of the housing base can be configured to be placed onto the skin (e.g., on the upper arm) of the subject. The planar portion can be provided surrounding a receiving portion **410.** An adhesive (not shown) can be placed on the planar portion of the housing base. The adhesive can create a seal on the skin that prevents the device from being removed from the skin without intentional removal from a user. An appropriate biocompatible adhesive material or gasket material can be placed on the planar portion on the housing base, to promote adhesion of the device onto the subject's skin for improved contact. Any suitable adhesive can be used. The adhesive can be a hydrogel, an acrylic, a polyurethane gel, a hydrocolloid, or a silicone gel.

The planar portion **405** may comprise a flexible base. The flexible base may conform to the shape of the skin of the user on which the patch mount is mounted. The flexible base may be a plastic, silicone, natural or synthetic rubber, thermoplastic polyurethane, nylon, and neoprene.

The receiving portion **410** may comprise a sufficiently stiff material to hold a transmitter module disposed therein. The transmitter portion may be attached to the receiving portion by clips, latches, snaps, straps, tethers, Velcro^{™}, tape, hook and loop, tack features, screw fasteners, tabs, magnetic fasteners, or any other suitable connection mechanisms such as elastic bands and adhesives. The receiving portion may be sized and shaped to receive a transmitter module **200** of the present disclosure. The receiving portion may have an opening which may facilitate contact between a sensor disposed on the patient facing surface of a transmitter module with the skin. In some cases, the receiving portion and the planar portion are disposable. In some cases, the receiving portion and the planar portion may be reused.

The planar portion may comprise an adhesive on the bottom (patent facing) surface. The adhesive can be a hydrogel. Optionally in any of the embodiments disclosed herein, the hydrogel can comprise a synthetic polymer, a natural polymer, a derivative thereof, or a combination thereof. Examples of synthetic polymers include, but are not limited to poly(acrylic acid), poly(vinyl alcohol) (PVA), poly(vinyl pyrrolidone) (PVP), poly (ethylene glycol) (PEG), and polyacrylamide. Examples of natural polymers include, but are not limited to alginate, cellulose, chitin, chitosan, dextran, hyaluronic acid, pectin, starch, xanthan gum, collagen, silk, keratin, elastin, resilin, gelatin, and agar.

In some embodiments, the adhesive can be pre-attached to the planar portion **405** on the on a skin facing side. The device can comprise a protective film or backing covering the adhesive on the planar portion. The protective film can be removed prior to use of the device and placement of the device on the subject's skin. In another embodiment, an adhesive in the form of a gel, a hydrogel, a paste, or a cream can be applied to skin of the subject or to the planar portion on the housing base of the device, prior to placement of the device on the subject's skin. The adhesive can then be placed in contact with the subject's skin for a predetermined amount of time (e.g., on the order of several seconds to several minutes) in order to form an adhesion layer between the skin and device. The adhesive can be a pressure-sensitive adhesive or a heat-sensitive adhesive. In some embodiments, the adhesive can be hypoallergenic.

In some embodiments, the adhesive can be a peelable adhesive, and can have a shape and size corresponding to the planar portion on the housing base of the device. In the example shown in **FIG. 12B****,** the planar portion on the housing base can be in the shape of an annular ring, although any shape can be contemplated. Accordingly, the peelable adhesive can be provided as an annular ring corresponding to the planar portion on the housing base. The skin facing side of a transmitter module may be exposed to a patient skin via an annular opening in the planar portion. In some embodiments and additional support may be placed between the planar portion and the adhesive. In some embodiments the planar portion may comprise a foam layer between the planar portion and the adhesive.

**FIG. 12C** illustrates a view of an integrated sensor patch system, in accordance with some embodiments. In the illustrated embodiment, a transmitter module **200** may be integrated with sensor **100** into a patch system of the present disclosure. An integrated sensor patch system may comprise integrated sensor and transmitter module **290.** An integrated sensor and transmitter module may be mounted on a planar portion **405,** which may comprise an adhesive also disposed thereon, as disclosed herein. **FIG. 12C** further illustrates a plurality of discrete sensor elements **105** which may be disposed on a top surface of an integrated sensor patch system. The illustrated embodiment may be attached to a connected device of the present disclosure, for example, as a breath sensing system.

**FIG. 13A, FIG. 13B, FIG. 13C,** and **FIG. 13D** illustrate an example of a patch system which may be coupled to a transmitter module which is also coupleable to an arm band, in accordance with some embodiments. **FIG. 13A** illustrates a top view of an example of a patch system which may be coupled to a transmitter module which is also coupleable to an arm band, in accordance with some embodiments. The planar portion **405** of the housing base can be configured to be placed onto the skin (e.g., on the upper arm) of the subject. The planar portion can be provided surrounding a receiving portion **410.** The receiving portion

As shown the patch system **400** may be smaller than the body part to be measured. The planar portion **405** may be circular, may be ellipsoid, may be rectangular, may be square, may be irregularly shaped, or may be any other shape. The planar portion may be cut to fit a shape which may allow movement of the tissue thereunder. The planar portion may be cut to a shape which may provide support to the tissue thereunder. The illustrated embodiment shows a circular planar portion with a diameter of 90 millimeters. The diameter of the planar portion may be less than 500 mm. The diameter of the planar portion may be less than 100 mm. The diameter of the planar portion may be less than 10 millimeters. The size of the planar portion may be sufficient to allow for coupling of a transmitter module of the present disclosure.

**FIG. 13B** illustrates a side view of an example of a patch system which may be coupled to a transmitter module which is also coupleable to an arm band, in accordance with some embodiments. As shown, the patch system may comprise a total height (e.g. a distance from the patient skin to the top of the device) of less than 10 mm. In some examples, the total height is less than 30 mm. In some examples, the total height is less than 5 mm. In some cases, the patch system comprises side wall which are high enough to clip the transmitter module within a patch system as disclosed herein.

**FIG. 13C** and **FIG. 13D** illustrate an example of a patch system coupled to a transmitter module which is also coupleable to an arm band, in accordance with some embodiments. The receiving portion **410** may comprise a sufficiently stiff material to hold a transmitter module disposed therein. The transmitter portion may be attached to the receiving portion by clips, latches, snaps, straps, tethers, Velcro^{™}, tape, hook and loop, tack features, screw fasteners, tabs, magnetic fasteners, or any other suitable connection mechanisms such as elastic bands and adhesives. The receiving portion may be sized and shaped to receive a transmitter module **200** of the present disclosure. The receiving portion may have an opening **415** which may facilitate contact between a sensor disposed on the patient facing surface of a transmitter module with the skin. In some cases, the receiving portion and the planar portion are disposable. In some cases, the receiving portion and the planar portion may be reused.

### CONNECTED DEVICE

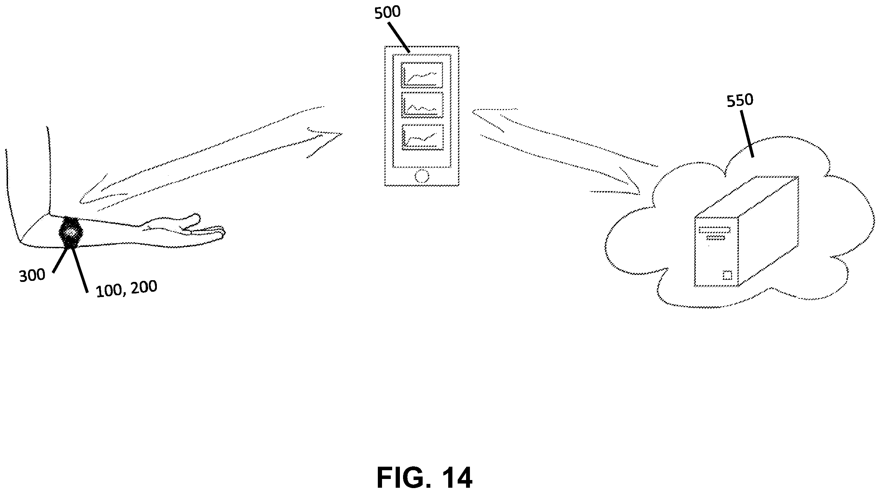

**FIG. 14** shows a wearable device of the present disclosure in connection with two connected devices, in accordance with some embodiments. In the illustrated embodiment, transmitter module **200** may receive and/or transmit data to a first connected device which is smartphone or tablet **500.** In the illustrated embodiment, smartphone or tablet **500** may receive and/or transmit data to a second connected device which is a remote server **550.** In some cases, a remote server may not be necessary. In some cases, a smartphone or tablet may not be necessary.

In some embodiments, the systems, devices, and methods described herein include one or more connected devices or use of the same. The connected device may be a digital processing device which may be connected wirelessly or by wired connection to devices and systems of the present disclosure. In some embodiments, the connected device may be optionally connected to a computer network. In further embodiments, the connected device is optionally connected to the Internet such that it accesses the World Wide Web. In still further embodiments, the connected device is optionally connected to a cloud computing infrastructure. In other embodiments, the connected device is optionally connected to an intranet. In other embodiments, the connected device is optionally connected to a data storage device. In other embodiments, the connected device is connected to a cellular data network. In still other embodiments, the connected device is connected via Bluetooth.

In accordance with the description herein, suitable connected devices include, by way of non-limiting examples, server computers, desktop computers, laptop computers, notebook computers, sub-notebook computers, netbook computers, netpad computers, set-top computers, media streaming devices, handheld computers, Internet appliances, mobile smartphones (e.g., Apple^{®} iPhone, Android-enabled device, Blackberry^{®}), tablet computers (e.g., Apple^{®} iPad, Samsung^{®} Galaxy Tab), personal digital assistants, video game consoles, and vehicles. Those of skill in the art will recognize that many smartphones are suitable for use in the system described herein. Those of skill in the art will also recognize that select televisions, video players, and digital music players with optional computer network connectivity are suitable for use in the system described herein. Suitable tablet computers include those with booklet, slate, and convertible configurations, known to those of skill in the art.

In accordance with the description herein, a connected device may be mobile device **500** such as a laptop, tablet, or smart phone. In some cases, the mobile device may be local to a user. A connected device that is a mobile device may be a mobile smartphone (e.g., Apple^{®} iPhone, Android-enabled device, Blackberry^{®}), a tablet computer (e.g., Apple^{®} iPad, Samsung^{®} Galaxy Tab), a desktop computer, a laptop computer, etc. The mobile device may allow a user control over the device.

In some cases, the mobile device may allow the user to access all or part of the sensor data. The mobile device may comprise a user interface. The mobile device may comprise software stored thereon which may allow a user to control functionality of the device. The software may comprise a mobile app. The software may comprise a browser plug-in. The software may comprise a standalone application. The software may allow a user to control functionality such as turning the device on or off, calibration, firmware updates, alerts, sensitivity, the number of type of analytes, set the sensor type, etc. In an example, the mobile device may display to a user a value of one or more analytes of the present disclosure (e.g. a concentration of an analyte). The mobile device may display the value of the analyte in substantially real time. The mobile device may display an upper and/or a lower threshold of the one or more analytes. For example, a patient may be able to see if a level of the analyte is too high or too low. In some cases, the user interface may prompt a user to change a behavior based on the data collected.

In accordance with the description herein, a connected device may be a remote server **550.** The remote server may be a cloud server. The remote server may store data. For example, the remote server may store data which does not need to be immediately available to a user. The remote server may store data when there is insufficient space to store the data on the mobile device and/or the transmitter module itself. The remote server may analyze data. The remote server may store data which may be analyzed by a third party such as a doctor, a personal trainer, a coach, etc.

### Computer program

In some embodiments, the platforms, systems, media, and methods disclosed herein include at least one computer program, or use of the same. A computer program includes a sequence of instructions, executable in the connected device's CPU, written to perform a specified task. Computer readable instructions may be implemented as program modules, such as functions, objects, Application Programming Interfaces (APIs), data structures, and the like, that perform particular tasks or implement particular abstract data types. In light of the disclosure provided herein, those of skill in the art will recognize that a computer program may be written in various versions of various languages.

The functionality of the computer readable instructions may be combined or distributed as desired in various environments. In some embodiments, a computer program comprises one sequence of instructions. In some embodiments, a computer program comprises a plurality of sequences of instructions. In some embodiments, a computer program is provided from one location. In other embodiments, a computer program is provided from a plurality of locations. In various embodiments, a computer program includes one or more software modules. In various embodiments, a computer program includes, in part or in whole, one or more web applications, one or more mobile applications, one or more standalone applications, one or more web browser plug-ins, extensions, add-ins, or add-ons, or combinations thereof.

### Mobile application

In some embodiments, a computer program includes a mobile application provided to a mobile connected device. In some embodiments, the mobile application is provided to a mobile connected device at the time it is manufactured. In other embodiments, the mobile application is provided to a mobile connected device via the computer network described herein.

In view of the disclosure provided herein, a mobile application is created by techniques known to those of skill in the art using hardware, languages, and development environments known to the art. Those of skill in the art will recognize that mobile applications are written in several languages. Suitable programming languages include, by way of non-limiting examples, C, C++, C#, Objective-C, Java^{™}, Javascript, Pascal, Object Pascal, Python^{™}, Ruby, VB.NET, WML, and XHTML/HTML with or without CSS, or combinations thereof.

Suitable mobile application development environments are available from several sources. Commercially available development environments include, by way of non-limiting examples, AirplaySDK, alcheMo, Appcelerator^{®}, Celsius, Bedrock, Flash Lite, .NET Compact Framework, Rhomobile, and WorkLight Mobile Platform. Other development environments are available without cost including, by way of non-limiting examples, Lazarus, MobiFlex, MoSync, and Phonegap. Also, mobile device manufacturers distribute software developer kits including, by way of non-limiting examples, iPhone and iPad (iOS) SDK, Android^{™} SDK, BlackBerry^{®} SDK, BREW SDK, Palm^{®} OS SDK, Symbian SDK, webOS SDK, and Windows^{®} Mobile SDK.

Those of skill in the art will recognize that several commercial forums are available for distribution of mobile applications including, by way of non-limiting examples, Apple^{®} App Store, Google^{®} Play, Chrome WebStore, BlackBerry^{®} App World, App Store for Palm devices, App Catalog for webOS, Windows^{®} Marketplace for Mobile, Ovi Store for Nokia^{®} devices, Samsung^{®} Apps, and Nintendo^{®} DSi Shop.

### Web application

In some embodiments, a computer program includes a web application. In light of the disclosure provided herein, those of skill in the art will recognize that a web application, in various embodiments, utilizes one or more software frameworks and one or more database systems. In some embodiments, a web application is created upon a software framework such as Microsoft^{®} .NET or Ruby on Rails (RoR). In some embodiments, a web application utilizes one or more database systems including, by way of non-limiting examples, relational, non-relational, object oriented, associative, and XML database systems. In further embodiments, suitable relational database systems include, by way of non-limiting examples, Microsoft^{®} SQL Server, mySQL^{™}, and Oracle^{®}. Those of skill in the art will also recognize that a web application, in various embodiments, is written in one or more versions of one or more languages. A web application may be written in one or more markup languages, presentation definition languages, client-side scripting languages, server-side coding languages, database query languages, or combinations thereof. In some embodiments, a web application is written to some extent in a markup language such as Hypertext Markup Language (HTML), Extensible Hypertext Markup Language (XHTML), or eXtensible Markup Language (XML). In some embodiments, a web application is written to some extent in a presentation definition language such as Cascading Style Sheets (CSS). In some embodiments, a web application is written to some extent in a client-side scripting language such as Asynchronous Javascript and XML (AJAX), Flash^{®} Actionscript, Javascript, or Silverlight^{®}. In some embodiments, a web application is written to some extent in a server-side coding language such as Active Server Pages (ASP), ColdFusion^{®}, Perl, Java^{™}, JavaServer Pages (JSP), Hypertext Preprocessor (PHP), Python^{™}, Ruby, Tcl, Smalltalk, WebDNA^{®}, or Groovy. In some embodiments, a web application is written to some extent in a database query language such as Structured Query Language (SQL). In some embodiments, a web application integrates enterprise server products such as IBM^{®} Lotus Domino^{®}. In some embodiments, a web application includes a media player element. In various further embodiments, a media player element utilizes one or more of many suitable multimedia technologies including, by way of non-limiting examples, Adobe^{®} Flash^{®}, HTML 5, Apple^{®} QuickTime^{®}, Microsoft^{®} Silverlight^{®}, Java^{™}, and Unity^{®}.

### Web browser plug-in

In some embodiments, the computer program includes a web browser plug-in (e.g., extension, etc.). In computing, a plug-in is one or more software components that add specific functionality to a larger software application. Makers of software applications support plug-ins to enable third-party developers to create abilities which extend an application, to support easily adding new features, and to reduce the size of an application. When supported, plug-ins enable customizing the functionality of a software application. For example, plug-ins are commonly used in web browsers to play video, generate interactivity, scan for viruses, and display particular file types. Those of skill in the art will be familiar with several web browser plug-ins including, Adobe^{®} Flash^{®} Player, Microsoft^{®} Silverlight^{®}, and Apple^{®} QuickTime^{®}. In some embodiments, the toolbar comprises one or more web browser extensions, add-ins, or add-ons. In some embodiments, the toolbar comprises one or more explorer bars, tool bands, or desk bands.

In view of the disclosure provided herein, those of skill in the art will recognize that several plug-in frameworks are available that enable development of plug-ins in various programming languages, including, by way of non-limiting examples, C++, Delphi, Java^{™}, PHP, Python^{™}, and VB .NET, or combinations thereof.

Web browsers (also called Internet browsers) are software applications, designed for use with network-connected devices, for retrieving, presenting, and traversing information resources on the World Wide Web. Suitable web browsers include, by way of non-limiting examples, Microsoft^{®} Internet Explorer^{®}, Mozilla^{®} Firefox^{®}, Google^{®} Chrome, Apple^{®} Safari^{®}, Opera Software^{®} Opera^{®}, and KDE Konqueror. In some embodiments, the web browser is a mobile web browser. Mobile web browsers (also called mircrobrowsers, mini-browsers, and wireless browsers) are designed for use on mobile connected devices including, by way of non-limiting examples, handheld computers, tablet computers, netbook computers, subnotebook computers, smartphones, music players, personal digital assistants (PDAs), and handheld video game systems. Suitable mobile web browsers include, by way of non-limiting examples, Google^{®} Android^{®} browser, RIM BlackBerry^{®} Browser, Apple^{®} Safari^{®}, Palm^{®} Blazer, Palm^{®} WebOS^{®} Browser, Mozilla^{®} Firefox^{®} for mobile, Microsoft^{®} Internet Explorer^{®} Mobile, Amazon^{®} Kindle^{®} Basic Web, Nokia^{®} Browser, Opera Software^{®} Opera^{®} Mobile, and Sony^{®} PSP^{™} browser.

### Standalone application

In some embodiments, a computer program includes a standalone application, which is a program that is run as an independent computer process, not an add-on to an existing process, e.g., not a plug-in. Those of skill in the art will recognize that standalone applications are often compiled. A compiler is a computer program(s) that transforms source code written in a programming language into binary object code such as assembly language or machine code. Suitable compiled programming languages include, by way of non-limiting examples, C, C++, Objective-C, COBOL, Delphi, Eiffel, Java^{™}, Lisp, Python^{™}, Visual Basic, and VB .NET, or combinations thereof. Compilation is often performed, at least in part, to create an executable program. In some embodiments, a computer program includes one or more executable complied applications.

### Software modules

In some embodiments, the platforms, systems, media, and methods disclosed herein include software, server, and/or database modules, or use of the same. In view of the disclosure provided herein, software modules are created by techniques known to those of skill in the art using machines, software, and languages known to the art. The software modules disclosed herein are implemented in a multitude of ways. In various embodiments, a software module comprises a file, a section of code, a programming object, a programming structure, or combinations thereof. In further various embodiments, a software module comprises a plurality of files, a plurality of sections of code, a plurality of programming objects, a plurality of programming structures, or combinations thereof. In various embodiments, the one or more software modules comprise, by way of non-limiting examples, a web application, a mobile application, and a standalone application. In some embodiments, software modules are in one computer program or application. In other embodiments, software modules are in more than one computer program or application. In some embodiments, software modules are hosted on one machine. In other embodiments, software modules are hosted on more than one machine. In further embodiments, software modules are hosted on cloud computing platforms. In some embodiments, software modules are hosted on one or more machines in one location. In other embodiments, software modules are hosted on one or more machines in more than one location.

### Processing Device

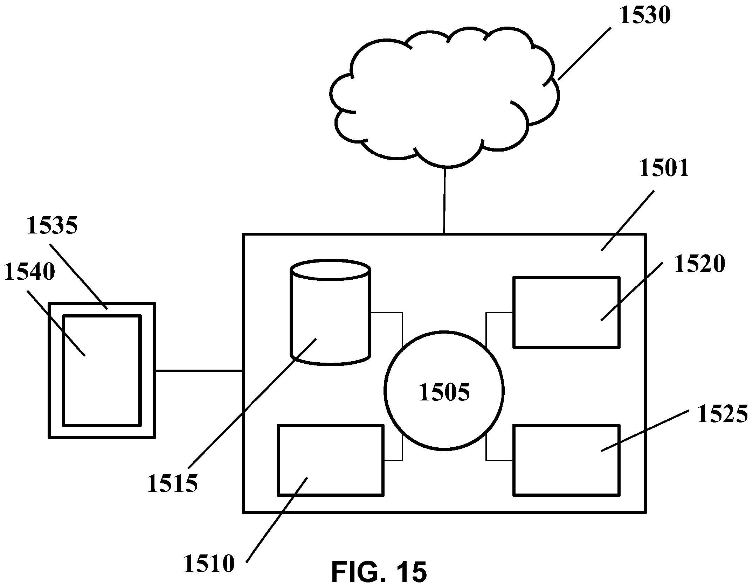

**FIG. 15** illustrates an example connected device **1501** programmed or otherwise configured to interface with a transmitter module **200,** in accordance with some embodiments. In further embodiments, the connected includes one or more hardware central processing units (CPUs), general purpose graphics processing units (GPGPUs), or field programmable gate arrays (FPGAs) that carry out the device's functions. In still further embodiments, the connected further comprises an operating system configured to perform executable instructions.

In some embodiments, the connected includes an operating system configured to perform executable instructions. The operating system is, for example, software, including programs and data, which manages the device's hardware and provides services for execution of applications. Those of skill in the art will recognize that suitable server operating systems include, by way of non-limiting examples, FreeBSD, OpenBSD, NetBSD^{®}, Linux, Apple^{®} Mac OS X Server^{®}, Oracle^{®} Solaris^{®}, Windows Server^{®}, and Novell^{®} NetWare^{®}. Those of skill in the art will recognize that suitable personal computer operating systems include, by way of non-limiting examples, Microsoft^{®} Windows^{®}, Apple^{®} Mac OS X^{®}, UNIX^{®}, and UNIX-like operating systems such as GNU/Linux^{®}. In some embodiments, the operating system is provided by cloud computing. Those of skill in the art will also recognize that suitable mobile smart phone operating systems include, by way of non-limiting examples, Nokia^{®} Symbian^{®} OS, Apple^{®} iOS^{®}, Research In Motion^{®} BlackBerry OS^{®}, Google^{®} Android^{®}, Microsoft^{®} Windows Phone^{®} OS, Microsoft^{®} Windows Mobile^{®} OS, Linux^{®}, and Palm^{®} WebOS^{®}. Those of skill in the art will also recognize that suitable media streaming device operating systems include, by way of non-limiting examples, Apple TV^{®}, Roku^{®}, Boxee^{®}, Google TV^{®}, Google Chromecast^{®}, Amazon Fire^{®}, and Samsung^{®} HomeSync^{®}. Those of skill in the art will also recognize that suitable video game console operating systems include, by way of non-limiting examples, Sony^{®} PS3^{®}, Sony^{®} PS4^{®}, Microsoft^{®} Xbox 360^{®}, Microsoft Xbox One, Nintendo^{®} Wii^{®}, Nintendo^{®} Wii U^{®}, Nintendo^{®} Switch^{®}, and Ouya^{®}.

In some embodiments, the device includes a storage and/or memory device. The storage and/or memory device is one or more physical apparatuses used to store data or programs on a temporary or permanent basis. In some embodiments, the device is volatile memory and requires power to maintain stored information. In some embodiments, the device is non-volatile memory and retains stored information when the connected is not powered. In further embodiments, the non-volatile memory comprises flash memory. In some embodiments, the non-volatile memory comprises dynamic random-access memory (DRAM). In some embodiments, the non-volatile memory comprises ferroelectric random access memory (FRAM). In some embodiments, the non-volatile memory comprises phase-change random access memory (PRAM). In other embodiments, the device is a storage device including, by way of non-limiting examples, CD-ROMs, DVDs, flash memory devices, magnetic disk drives, magnetic tapes drives, optical disk drives, and cloud computing based storage. In further embodiments, the storage and/or memory device is a combination of devices such as those disclosed herein.

In some embodiments, the connected includes a display to send visual information to a user. In some embodiments, the display is a cathode ray tube (CRT). In some embodiments, the display is a liquid crystal display (LCD). In further embodiments, the display is a thin film transistor liquid crystal display (TFT-LCD). In some embodiments, the display is an organic light emitting diode (OLED) display. In various further embodiments, on OLED display is a passive-matrix OLED (PMOLED) or active-matrix OLED (AMOLED) display. In some embodiments, the display is a plasma display. In other embodiments, the display is a video projector. In still further embodiments, the display is a combination of devices such as those disclosed herein.

In some embodiments, the connected includes an input device to receive information from a user. In some embodiments, the input device is a keyboard. In some embodiments, the input device is a pointing device including, by way of non-limiting examples, a mouse, trackball, track pad, joystick, game controller, or stylus. In some embodiments, the input device is a touch screen or a multi-touch screen. In other embodiments, the input device is a microphone to capture voice or other sound input. In other embodiments, the input device is a video camera or other sensor to capture motion or visual input. In further embodiments, the input device is a Kinect, Leap Motion, or the like. In still further embodiments, the input device is a combination of devices such as those disclosed herein.

Referring again to **FIG. 15****,** an exemplary connected device **1501** is programmed or otherwise configured to connect to a transmitter module as described herein. The device **1501** can regulate various aspects of the transmitter module **200** of the present disclosure, such as, for example, performing processing data, storing data, turning the device on and off, syncing data to an external server, etc. In this embodiment, the connected device **1501** includes a central processing unit (CPU, also "processor" and "computer processor" herein) **1505,** which can be a single core or multi core processor, or a plurality of processors for parallel processing. The connected device **1501** also includes memory or memory location **1510** (e.g., random-access memory, read-only memory, flash memory), electronic storage unit **1515** (e.g., hard disk), communication interface **1520** (e.g., network adapter, Bluetooth radio, etc.) for communicating with one or more other systems, and peripheral devices **1525,** such as cache, other memory, data storage and/or electronic display adapters. The memory **1510,** storage unit **1515,** interface **1520** and peripheral devices **1525** are in communication with the CPU **1505** through a communication bus (solid lines), such as a motherboard. The storage unit **1515** can be a data storage unit (or data repository) for storing data.

The connected device **1501** can be operatively coupled to a computer network ("network") **1530** with the aid of the communication interface **1520.** The network **1530** can be the Internet, an internet and/or extranet, or an intranet and/or extranet that is in communication with the Internet. The network **1530** in some cases is a telecommunication and/or data network. The network **1530** can include one or more computer servers, which can enable distributed computing, such as cloud computing. The network **1530,** in some cases with the aid of the device **1501,** can implement a peer-to-peer network, which may enable devices coupled to the device **1501** to behave as a client or a server.

Continuing to refer to **FIG. 15****,** the CPU **1505** can execute a sequence of machine-readable instructions, which can be embodied in a program or software. The instructions may be stored in a memory location, such as the memory **1510.** The instructions can be directed to the CPU **1505,** which can subsequently program or otherwise configure the CPU **1505** to implement methods of the present disclosure. Examples of operations performed by the CPU **1505** can include fetch, decode, execute, and write back. The CPU **1505** can be part of a circuit, such as an integrated circuit. One or more other components of the device **1501** can be included in the circuit. In some cases, the circuit is an application specific integrated circuit (ASIC) or a field programmable gate array (FPGA).

Continuing to refer to **FIG. 15****,** the storage unit **1515** can store files, such as drivers, libraries and saved programs. The storage unit **1515** can store user data, e.g., user preferences and user programs. The connected device **1501** in some cases can include one or more additional data storage units that are external, such as located on a remote server that is in communication through an intranet or the Internet.

Continuing to refer to **FIG. 15****,** the connected device **1501** can communicate with one or more remote computer systems through the network **1530.** For instance, the device **1501** can communicate with a remote computer system of a user. Examples of remote computer systems include personal computers (e.g., portable PC), slate or tablet PCs (e.g., Apple^{®} iPad, Samsung^{®} Galaxy Tab), telephones, Smart phones (e.g., Apple^{®} iPhone, Android-enabled device, Blackberry^{®}), or personal digital assistants.

Methods as described herein can be implemented by way of machine (e.g., computer processor) executable code stored on an electronic storage location of the connected device **1501,** such as, for example, on the memory **1510** or electronic storage unit **1515.** The machine executable or machine readable code can be provided in the form of software. During use, the code can be executed by the processor **1505.** In some cases, the code can be retrieved from the storage unit **1515** and stored on the memory **1510** for ready access by the processor **1505.** In some situations, the electronic storage unit **1515** can be precluded, and machine-executable instructions are stored on memory **1510.**

### Non-transitory computer readable storage medium

In some embodiments, the platforms, systems, media, and methods disclosed herein include one or more non-transitory computer readable storage media encoded with a program including instructions executable by the operating system of an optionally networked connected device. In further embodiments, a computer readable storage medium is a tangible component of a connected device. In still further embodiments, a computer readable storage medium is optionally removable from a connected device. In some embodiments, a computer readable storage medium includes, by way of non-limiting examples, CD-ROMs, DVDs, flash memory devices, solid state memory, magnetic disk drives, magnetic tape drives, optical disk drives, cloud computing systems and services, and the like. In some cases, the program and instructions are permanently, substantially permanently, semi-permanently, or non-transitorily encoded on the media.

The processes explained above are described in terms of computer software and hardware. The techniques described may constitute machine-executable instructions embodied within a tangible or non-transitory machine (e.g., computer) readable storage medium, that when executed by a machine will cause the machine to perform the operations described. Additionally, the processes may be embodied within hardware, such as an application specific integrated circuit ("ASIC") or otherwise.

A tangible non-transitory machine-readable storage medium includes any mechanism that provides (i.e., stores) information in a form accessible by a machine (e.g., a computer, network device, personal digital assistant, manufacturing tool, any device with a set of one or more processors, etc.). For example, a machine-readable storage medium includes recordable/non-recordable media (e.g., read only memory (ROM), random access memory (RAM), magnetic disk storage media, optical storage media, flash memory devices, etc.).

### DOCKING STATION

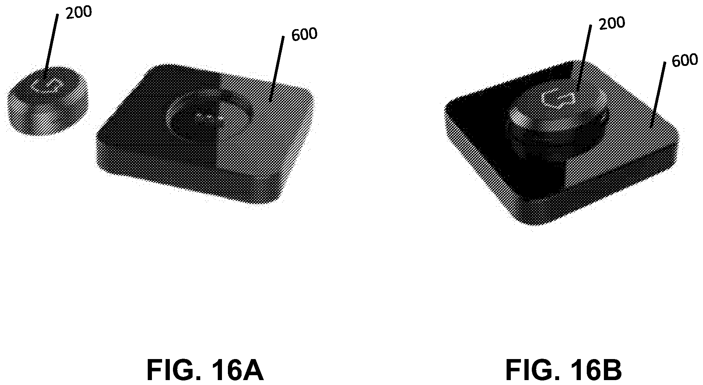

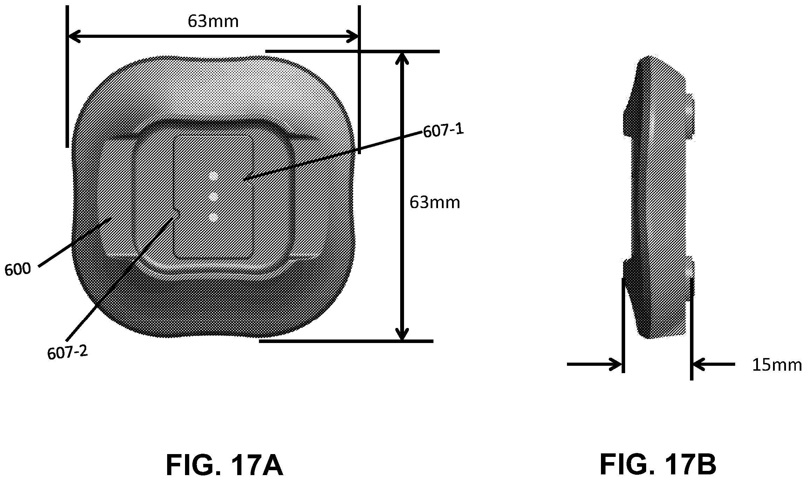

**FIG. 16A, FIG. 16B****,** **FIG. 17A,** and **FIG. 17B** illustrate a docking station **600,** which may be coupled to a transmitter module **200,** in accordance with some embodiments. The transmitter module may comprise an embodiment, variation, or example of a transmitter module 200 described elsewhere herein.

**FIG. 16A** shows a transmitter module decoupled from a docking station, in accordance with some embodiments. **FIG. 16B** shows a transmitter module coupled from a docking station, in accordance with some embodiments. As shown, the transmitter may be freely uncoupled and coupled to a docking station, which may provide convenient ways of charging the transmitter. Optionally, the transmitter may be charged via wires, or wirelessly. Optionally, the docking station may also be utilized to provide an interface for coupling the transmitter to an external computer or to upload data of the transmitter to a database. The docking station may comprise one or more to connect the docking station to a wall socket. The docking station may comprise one or more ports to connect the docking station to a computer. The docking station may be charged and/or connected to an external device by USB.

**FIG. 17A** shows a transmitter module decoupled from a docking station, in accordance with some embodiments. **FIG. 17B** shows a transmitter module coupled from a docking station, in accordance with some embodiments. As shown, the transmitter may be freely uncoupled and coupled to a docking station, which may provide convenient ways of charging the transmitter. Optionally, the transmitter may be charged via wires, or wirelessly. Optionally, the docking station may also be utilized to provide an interface for coupling the transmitter to an external computer or to upload data of the transmitter to a database. The docking station may comprise one or more to connect the docking station to a wall socket. The docking station may comprise one or more ports to connect the docking station to a computer. The docking station may be charged and/or connected to an external device by USB.

While preferred embodiments have been shown and described herein, it will be obvious to those skilled in the art that such embodiments are provided by way of example only. The invention is defined by the appended claims.

## Claims

1. A modular sensor (100) comprising:
a substrate (101);
a plurality of contact electrodes (109) provided on a surface of the substrate (100); and
a plurality of sensing areas disposed between the plurality of contact electrodes (109) to collectively form a plurality of sensor elements (105), wherein each sensor element (105) of the plurality of sensor elements (105) comprises at least one sensing area extending longitudinally between and electrically connecting a pair of contact electrodes (103), wherein at least one of the plurality of sensor elements (105) comprises a sensing area comprising a nanoscale material extending longitudinally between and electrically connecting the pair of contact electrodes (109),
wherein the modular sensor (100) is configured to be operably and releasably coupled to a device (200) for use as a sensing apparatus.

2. The modular sensor (100) of claim 1, wherein the modular sensor (100) is configured to function as an active sensing unit when electronically coupled to the device (200).

3. The modular sensor (100) of claim 1, wherein the modular sensor (100) is configured to fit within a recessed housing on the device, and, optionally, wherein the modular sensor (100) is protected by the recessed housing.

4. The modular sensor of claim 1, wherein the substrate (101) comprises a ferrous metal or alloy, and the device comprises a magnetic material, and, optionally, wherein the modular sensor (100) is configured to be coupled and held in place on the device via an attractive force between the magnetic material and the ferrous metal or alloy.

5. The modular sensor (100) of claim 1, wherein the nanoscale material comprises graphene.

6. The modular sensor (100) of claim 1, wherein the sensing area of the plurality of sensor elements (105) each comprises a nanoscale material comprising graphene.

7. The modular sensor (100) of claim 1, wherein the plurality of sensor elements (105) is configured to detect one or more markers in a fluid, and, optionally, wherein the one or more markers are one or more biomarkers and wherein the fluid is a biological fluid of a subject.

8. The modular sensor (100) of claim 1, wherein the modular sensor (100) is configured to be operably and releasably coupled to the device without the use of tools, and, optionally, wherein the modular sensor (100) is configured to be operably and releasably coupled to the device in less than 10 seconds.

9. The modular sensor (100) of claim 8 wherein each of the plurality of sensor elements (105) is configured to detect a same biomarker.

10. The modular sensor (100) of claim 8, wherein the plurality of sensor elements (105) is configured operate in a multichannel multiplexed configuration, and, optionally, wherein each of the plurality of sensor elements (105-1, 105-2, 105-3) is configured to detect a different biomarker.

11. The modular sensor (100) of claim 8, wherein the one or more biomarkers comprises an electrolyte, glucose, lactic acid, IL6, a cytokine, HER2, Cortisol, ZAG, cholesterol, vitamins, a protein, a drug molecule, a metabolite, a peptide, an amino acid, a DNA, an RNA, an aptamer, an enzyme, a biomolecule, a chemical molecule, a synthetic molecule, or combinations thereof.

12. The modular sensor (100) of claim 8, wherein the biological fluid sample comprises sweat, breath, saliva, earwax, urine, semen, blood plasma, a bio-fluid, a chemical fluid, an air sample, a gas sample, or a combination thereof, and, optionally, wherein the biological fluid comprises sweat or interstitial fluid obtained via the surface of the skin or wherein the biological fluid comprises breath or lung originated water vapor obtained from exhaling on the device.

13. The modular sensor (100) of claim 8, wherein the plurality of sensor elements (105) is configured to detect the one or more biomarkers when in contact with the biological fluid sample.

14. The modular sensor (100) of claim 8, wherein the plurality of sensor elements (105) is capable of detecting the one or more biomarkers in a non-invasive manner, without requiring penetration of the subject's skin to extract the biological fluid sample.

15. The modular sensor (100) of claim 8, wherein the plurality of sensor elements (105) is configured to detect a presence and concentration of the one or more biomarkers substantially in real-time when the device is being worn on the subject or in proximity to the subject, and, optionally, wherein data indicative of the presence and concentrations of the one or more biomarkers is collected and stored by the device over a time period that the device is being worn on the subject or in proximity to the subject.

## Patentansprüche

1. Modularer Sensor (100), umfassend:
ein Substrat (101);
eine Vielzahl von Kontaktelektroden (109), die auf einer Oberfläche des Substrats (100) vorgesehen sind; und
eine Vielzahl von Abfühlbereichen, die zwischen der Vielzahl von Kontaktelektroden (109) angeordnet sind, um zusammen eine Vielzahl von Sensorelementen (105) auszubilden, wobei jedes Sensorelement (105) aus der Vielzahl von Sensorelementen (105) zumindest einen Abfühlbereich umfasst, der sich longitudinal zwischen einem Paar von Kontaktelektroden (103) erstreckt und dieses elektrisch verbindet, wobei zumindest eines aus der Vielzahl von Sensorelementen (105) einen Abfühlbereich umfasst, der ein Nanomaßstab-Material umfasst, das sich longitudinal zwischen dem Paar von Kontaktelektroden (109) erstreckt und dieses elektrisch verbindet,
wobei der modulare Sensor (100) ausgelegt ist, um mit einer Vorrichtung (200) zur Verwendung als Abfühlvorrichtung lösbar wirkverbunden zu sein.

2. Modularer Sensor (100) nach Anspruch 1, wobei der modulare Sensor (100) ausgelegt ist, um als eine aktive Abfühleinheit zu fungieren, wenn sie elektronisch mit der Vorrichtung (200) gekoppelt ist.

3. Modularer Sensor (100) nach Anspruch 1, wobei der modulare Sensor (100) ausgelegt ist, um innerhalb eines vertieften Gehäuses auf der Vorrichtung zu passen und wobei der modulare Sensor (100) gegebenenfalls durch das vertiefte Gehäuse geschützt ist.

4. Modularer Sensor nach Anspruch 1, wobei das Substrat (101) ein eisenhältiges Material oder eine Legierung umfasst und die Vorrichtung ein magnetisches Material umfasst und wobei der modulare Sensor (100) gegebenenfalls ausgelegt ist, um über eine Anziehungskraft zwischen dem magnetischen Material und dem eisenhältigen Metall oder Legierung auf der Vorrichtung gekoppelt und in Position gehalten zu werden.

5. Modularer Sensor (100) nach Anspruch 1, wobei das Nanomaßstab-Material Graphen umfasst.

6. Modularer Sensor (100) nach Anspruch 1, wobei der Abfühlbereich der Vielzahl von Sensorelementen (105) jeweils ein Nanomaßstab-Material, umfassend Graphen, umfasst.

7. Modularer Sensor (100) nach Anspruch 1, wobei die Vielzahl von Sensorelementen (105) ausgelegt ist, um einen oder mehrere Marker in einem Fluid zu detektieren, wobei der eine oder die mehreren Marker gegebenenfalls ein oder mehrere Biomarker sind und wobei das Fluid ein biologisches Fluid eines Individuums ist.

8. Modularer Sensor (100) nach Anspruch 1, wobei der modulare Sensor (100) ausgelegt ist, um ohne Verwendung von Werkzeug lösbar mit der Vorrichtung wirkverbunden zu sein und wobei der modulare Sensor (100) gegebenenfalls ausgelegt ist, um in weniger als 10 s lösbar mit der Vorrichtung wirkverbunden zu sein.

9. Modularer Sensor (100) nach Anspruch 8, wobei jedes aus der Vielzahl von Sensorelementen (105) ausgelegt ist, um denselben Biomarker zu detektieren.

10. Modularer Sensor (100) nach Anspruch 8, wobei die Vielzahl von Sensorelementen (105) ausgelegt ist, um in einer Mehrkanal-Multiplex-Konfiguration betrieben zu werden und wobei jedes aus der Vielzahl von Sensorelementen (105-1, 105-2, 105-3) gegebenenfalls ausgelegt ist, um einen anderen Biomarker zu detektieren.

11. Modularer Sensor (100) nach Anspruch 8, wobei der eine oder die mehreren Biomarker einen Elektrolyten, Glucose, Milchsäure, IL6, ein Zytokin, HER2, Cortisol, ZAG, Cholesterin, Vitamine, ein Protein, ein Arzneimittelmolekül, einen Metaboliten, ein Peptid, eine Aminosäure, eine DNA, eine RNA, ein Aptamer, ein Enzym, ein Biomolekül, ein chemisches Molekül, ein synthetisches Molekül oder Kombinationen davon umfassen.

12. Modularer Sensor (100) nach Anspruch 8, wobei die biologische Fluidprobe Schweiß, Atemluft, Speichel, Ohrenschmalz, Urin, Sperma, Blutplasma, ein Biofluid, ein chemisches Fluid, eine Luftprobe, eine Gasprobe oder Kombinationen davon umfasst und wobei das biologische Fluid gegebenenfalls Schweiß oder Interstitialfluid umfasst, der/das über die Hautoberfläche erhalten wurde, oder wobei das biologische Fluid Atemluft oder Wasserdampf aus der Lunge, die/der durch Ausatmen auf die Vorrichtung erhalten wurde, umfasst.

13. Modularer Sensor (100) nach Anspruch 8, wobei die Vielzahl von Sensorelementen (105) ausgelegt ist, um den einen oder die mehreren Biomarker zu detektieren, wenn diese mit der biologischen Fluidprobe in Kontakt stehen.

14. Modularer Sensor (100) nach Anspruch 8, wobei die Vielzahl von Sensorelementen (105) dazu in der Lage ist, den einen oder die mehreren Biomarker auf nichtinvasive Weise zu detektieren, ohne dafür ein Durchstechen der Haut des Individuums zum Extrahieren der biologischen Fluidprobe zu benötigen.

15. Modularer Sensor (100) nach Anspruch 8, wobei die Vielzahl von Sensorelementen (105) ausgelegt ist, um ein Vorhandensein und eine Konzentration des einen oder der mehreren Biomarker im Wesentlichen in Echtzeit zu detektieren, wenn die Vorrichtung auf dem Individuum oder in der Nähe des Individuums getragen wird, und wobei Daten, die die Gegenwart und Konzentrationen des einen oder der mehreren Biomarker angeben, gegebenenfalls von der Vorrichtung über einen Zeitraum gesammelt und gespeichert werden, während dessen die Vorrichtung auf dem Individuum oder in der Nähe des Individuums getragen wird.

## Revendications

1. Capteur modulaire (100), comprenant :
un substrat (101) ;
une pluralité d'électrodes de contact (109) prévues sur une surface du substrat (100) ; et
une pluralité de zones de détection disposées entre la pluralité d'électrodes de contact (109) pour former collectivement une pluralité d'éléments de capteur (105), dans lequel chaque élément de capteur (105) de la pluralité d'éléments de capteur (105) comprend au moins une zone de détection s'étendant longitudinalement entre une paire d'électrodes de contact (103) et les connectant électriquement, dans lequel au moins un de la pluralité d'éléments de capteur (105) comprend une zone de détection comprenant un matériau à l'échelle nanométrique s'étendant longitudinalement entre la paire d'électrodes de contact (109) et les connectant électriquement,
dans lequel le capteur modulaire (100) est configuré pour être couplé de manière fonctionnelle et libérable à un dispositif (200) destiné à être utilisé en tant qu'appareil de détection.

2. Capteur modulaire (100) selon la revendication 1, dans lequel le capteur modulaire (100) est configuré pour fonctionner comme une unité de détection active lorsqu'il est couplé électroniquement au dispositif (200).

3. Capteur modulaire (100) selon la revendication 1, dans lequel le capteur modulaire (100) est configuré pour s'adapter à l'intérieur d'un logement en retrait sur le dispositif, et, facultativement, dans lequel le capteur modulaire (100) est protégé par le logement en retrait.

4. Capteur modulaire selon la revendication 1, dans lequel le substrat (101) comprend un métal ou un alliage ferreux, et le dispositif comprend un matériau magnétique, et facultativement dans lequel le capteur modulaire (100) est configuré pour être couplé à et maintenu en place sur le dispositif via une force d'attraction entre le matériau magnétique et le métal ou alliage ferreux.

5. Capteur modulaire (100) selon la revendication 1, dans lequel le matériau à l'échelle nanométrique comprend du graphène.

6. Capteur modulaire (100) selon la revendication 1, dans lequel la zone de détection de chacun de la pluralité d'éléments de capteur (105) comprend un matériau à l'échelle nanométrique comprenant du graphène.

7. Capteur modulaire (100) selon la revendication 1, dans lequel la pluralité d'éléments de capteur (105) est configurée pour détecter un ou plusieurs marqueurs dans un fluide, et facultativement dans lequel les un ou plusieurs marqueurs sont un ou plusieurs biomarqueurs et dans lequel le fluide est un fluide biologique d'un sujet.

8. Capteur modulaire (100) selon la revendication 1, dans lequel le capteur modulaire (100) est configuré pour être couplé de manière fonctionnelle et libérable au dispositif sans utiliser d'outils, et facultativement dans lequel le capteur modulaire (100) est configuré pour être couplé de manière fonctionnelle et libérable au dispositif en moins de 10 secondes.

9. Capteur modulaire (100) selon la revendication 8, dans lequel chacun de la pluralité d'éléments de capteur (105) est configuré pour détecter un même biomarqueur.

10. Capteur modulaire (100) selon la revendication 8, dans lequel la pluralité d'éléments de capteur (105) est configurée pour fonctionner dans une configuration multiplexée multicanal, et facultativement dans lequel chacun de la pluralité d'éléments de capteur (105-1, 105-2, 105-3) est configuré pour détecter un biomarqueur différent.

11. Capteur modulaire (100) selon la revendication 8, dans lequel les un ou plusieurs biomarqueurs comprennent un électrolyte, du glucose, de l'acide lactique, de l'IL6, une cytokine, HER2, du Cortisol, du ZAG, du cholestérol, des vitamines, une protéine, une molécule de médicament, un métabolite, un peptide, un acide aminé, un ADN, un ARN, un aptamère, une enzyme, une biomolécule, une molécule chimique, une molécule synthétique, ou des combinaisons de ceux-ci.

12. Capteur modulaire (100) selon la revendication 8, dans lequel l'échantillon de fluide biologique comprend de la sueur, de l'haleine, de la salive, du cérumen, de l'urine, du sperme, du plasma sanguin, un bio-fluide, un fluide chimique, un échantillon d'air, un échantillon de gaz ou une combinaison de ceux-ci, et facultativement dans lequel le fluide biologique comprend de la sueur ou un fluide interstitiel obtenu (e) via la surface de la peau ou dans lequel le fluide biologique comprend de la vapeur d'eau provenant de l'haleine ou des poumons obtenue à partir de l'expiration sur le dispositif.

13. Capteur modulaire (100) selon la revendication 8, dans lequel la pluralité d'éléments de capteur (105) est configurée pour détecter les un ou plusieurs biomarqueurs lorsqu'ils sont en contact avec l'échantillon de fluide biologique.

14. Capteur modulaire (100) selon la revendication 8, dans lequel la pluralité d'éléments de capteur (105) est capable de détecter les un ou plusieurs biomarqueurs d'une manière non invasive, sans nécessiter de pénétration de la peau du sujet pour extraire l'échantillon de fluide biologique.

15. Capteur modulaire (100) selon la revendication 8, dans lequel la pluralité d'éléments de capteur (105) est configurée pour détecter une présence et une concentration des un ou plusieurs biomarqueurs sensiblement en temps réel lorsque le dispositif est porté sur le sujet ou à proximité du sujet, et facultativement dans lequel des données indicatives de la présence et des concentrations des un ou plusieurs biomarqueurs sont collectées et stockées par le dispositif sur une période de temps pendant laquelle le dispositif est porté sur le sujet ou à proximité du sujet.
